# EUROPEAN PATENT SPECIFICATION

(11) **EP 1 004 671 B1**
(45) Date of publication and mention of the grant of the patent: **11.01.2006**
(21) Application number: 98936705.7
(22) Date of filing: 07.08.1998
(51) Int. Cl.: C12N 15/53, C12N 1/21, C12P 13/14, C12N 9/06, C12R 1/15

(54) **PROCESS FOR PRODUCING L-GLUTAMIC ACID BY FERMENTATION METHOD**
VERFAHREN FÜR DIE PRODUKTION VON L-GLUTAMINSÄURE MITTELS FERMENTATION
PROCEDE DE PRODUCTION D'ACIDE L-GLUTAMIQUE PAR FERMENTATION

(30) Priority: 12.08.1997 JP 21690697
(43) Date of publication of application: 31.05.2000
(73) Proprietor: Ajinomoto Co., Inc., Tokyo 104-0031 (JP)
(72) Inventor: KANNO, Souhei Ajinomoto Co., Inc., Kanagawa 210-8681 (JP); KIMURA, Eiichiro Ajinomoto Co., Inc., Kanagawa 210-8681 (JP); MATSUI, Kazuhiko Ajinomoto Co., Inc., Kanagawa 210-8681 (JP); OSUMI, Tsuyoshi Ajinomoto Co., Inc., Kanagawa 210-8681 (JP); NAKAMATSU, Tsuyoshi Ajinomoto Co., Inc., Kanagawa 210-8681 (JP)
(74) Representative: Strehl Schübel-Hopf & Partner
(86) International application number: PCT/JP1998/003535
(87) International publication number: WO 1999/007853

(56) References cited:
- FR-A- 2 575 492
- JP-A- 5 007 491
- KIM I-J ET AL: "GENETIC REGULATION FOR THE BIOSYNTHESIS OF GLUTAMATE FAMILY IN CORYNEBACTERIUM GLUTAMICUM" SANNEB MISAINMURHAG HOIJI - KOREAN JOURNAL OF APPLIED MICROBIOLOGY AND BIOENGINEERING, SEOUL, KR, vol. 14, no. 5, 1986, pages 427-432, XP000872380 ISSN: 0257-2389
- VANDECASTEELE J P ET AL: "Pathways and regulation of glutamate synthesis in a Corynebacterium sp. overproducing glutamate" JOURNAL OF GENERAL MICROBIOLOGY, vol. 90, 1975, pages 178-180, XP001070362
- ERTAN H: "THE EFFECT OF VARIOUS CULTURE CONDITIONS ON THE LEVELS OF AMMONIA ASSIMILATORY ENZYMES OF CORYNEBACTERIUM CALLUNAE" ARCHIVES OF MICROBIOLOGY, BERLIN, DE, vol. 1, no. 158, 1992, pages 42-47, XP001069261 ISSN: 0302-8933
- DEANE S M ET AL: "Cloning and sequencing of the gene for the Thiobacillus ferroxidans ATCC33020 glutamate synthase (GOGAT) small subunit and complementation of an Escherichia coli gltD mutant" GENE: AN INTERNATIONAL JOURNAL ON GENES AND GENOMES, ELSEVIER SCIENCE PUBLISHERS, BARKING, GB, vol. 177, no. 1, 24 October 1996 (1996-10-24), pages 261-263, XP004043406 ISSN: 0378-1119
- JONGSAREEJIT B ET AL : "Gene cloning, sequencing and enzymatic properties of glutamate synthase from the hyperthermophilic archaeon Pyrococcus sp. KOD1" MOL. GEN. GENET., vol. 254, no. 6, May 1997 (1997-05), pages 635-42, XP001079429
- GREGERSON RG ET AL: "Molecular characterization of NADH-dependent glutamate synthase from alfalfa nodules" PLANT CELL, vol. 5, no. 2, February 1993 (1993-02), pages 215-26, XP001078896
- BOERMANN-EL KHOLY ELKE R ET AL: "Glutamate dehydrogenase is not essential for glutamate formation by Corynebacterium glutamicum." 1993 , APPLIED AND ENVIRONMENTAL MICROBIOLOGY, VOL. 59, NR. 7, PAGE(S) 2329-2331 XP001079206 ISSN: 0099-2240 * abstract *
- J. BIOL. CHEM., Vol. 268(5), (1993), R. PELANDA et al., "Glutamate Synthase Genes of the Diazotroph Azospirillum Brasilense", p. 3099-3106, XP002914509
- GENE, Vol. 60, (1987), G. OLIVER et al., "Determination of the Nucleotide Sequence for the Glutamate Synthase Structural Genes of Escherichia Coli K-12", p. 1-11, XP002914510
- YEAST, Vol. 12, (1996), P. BALLARIO et al., "Yeast Sequencing Reports", p. 1359-1366, XP002914511
- AGRIC. BIOL. CHEM., Vol. 51, (1987), "Threonin Production by Coexistence of Clones Genes Coding Homoserine Dehydrogenase and Homoserine Kinase in Brevibacterium Lactofermentum", p. 93-100, XP002914512
- ARCH. MICROBIOL., Vol. 158(1), (1992), ERTAN H., "Some Properties of Glutamate Dehydrogenase, Glutamine Synthetase and Glutamate Synthase from Corynebacterium-Callunae", p. 35-41, XP002914513
- J. FERMENT. TECHNOL., Vol. 62(6), (1984), SUNG H.-C. et al., "Properties of Glutamate Synthase from Brevibacterium Flavum", p. 569-575, XP002914514
- J. FERMENT. TECHNOL., Vol. 62(4), (1984), SUNG H.-C. et al., "Production and Preparation of Glutamate Synthase EC-1.4.1.13 from Brevibacterium-Flavum", pages 371-376, XP002914515
- G. BECKERS ET AL.: "Glutamate synthase of Corynebacterium glutamicum is not essential for glutamate synthesis and is regulated by the nitrogen status." MICROBIOLOGY, vol. 147, 2001, pages 2961-2970,

## Description

### Technical Field

The present invention relates to a method for producing L-glutamic acid by fermentation. L-glutamic acid is an important amino acid which is used for food, clinical drugs and others.

### Background Art

Conventionally, L-glutamic acid has been mainly produced by fermentative methods using so-called L-glutamic acid-producing coryneform bacteria which belong to the genus Brevibacterium, Corynebacterium or Microbacterium, or mutants thereof (Amino Acid Fermentation, Gakkai Shuppan Center, 195-215, (1986)). As fermentative methods for producing L-glutamic acid using other bacterial strains, those which use microorganisms including Bacillus, Streptomyces, and Penicillium (U.S. Patent 3,220,929), and those which use microorganisms including the genera Pseudomonas, Arthrobacter, Serratia, and Candida (U.S. Patent 3,563,857) are known. Although productivity for L-glutamic acid has been much increased by using conventional methods, development of a method for producing L-glutamic acid which is more effective and inexpensive is required in order to satisfy further increasing demand.

L-glutamic acid is biosynthesized from α-ketoglutaric acid which is an intermediate in the citric acid cycle existing in cell of microorganisms, and there are two different biosynthetic pathways to form L-glutamic acid from α-ketoglutaric acid by assimilation of ammonium ion. One is the pathway through which, in the presence of ammonium ion with a high concentration, L-glutamic acid is synthesized by catalysis of glutamate dehydrogenase (hereinafter referred to as "GDH"), and the other is the pathway (GS/GOGAT pathway) through which L-glutamic acid is synthesized by glutamine synthetase (hereinafter referred to as "GS") which catalyzes reaction from L-glutamic acid and ammonium ion to glutamine, and by glutamine-oxoglutaric acid amino transferase (also called as "glutamate synthase", and hereinafter referred to as "GOGAT") which catalyzes L-glutamic acid synthetic reaction in which two molecules of L-glutamic acid is produced from one molecule of glutamine, that has been synthesized by GS, and one molecule of α-ketoglutaric acid. So far, L-glutamic acid production by a bacterial strain GDH pathway of which has been enhanced through elevation of the GDH activity has been reported, but L-glutamic acid production by a bacterial strain GS/GOGAT pathway of which is enhanced has not been known.

### Disclosure of the Invention

An object of the present invention is to breed bacterial strains having high L-glutamic acid productivity, and to provide a method for producing L-glutamic acid which is more effective and inexpensive, in order to satisfy further increasing demand of L-glutamic acid.

The present inventors has conducted studies on methods for producing L-glutamic acid using bacterial strains being enhanced GOGAT activity which is one of enzymes that participate in the GS/GOGAT pathway, and catalyzes production of L-glutamic acid. As a result, the inventors has found that a bactrerial strain having an ability to produce L-glutamic acid which is enhanced GOGAT activity has higher L-glutamic acid productivity, and thus completed the present invention.

That is, the present invention provides a bacterial strain belonging to the genus Corynebacterium which has an ability to produce L-glutamic acid, wherein glutamine-oxoglutarate amino transferase activity is enhanced in a cell of the bacterial strain. The enhancement of the glutamine-oxoglutarate amino transferase activity may be caused through amplifying the copy number of a gene encoding glutamine-oxoglutarate amino transferase. Alternatively, the enhancement of the glutamine-oxoglutarate amino transferase activity may be also caused through alteration of expression regulation sequence of a gene encoding the enzyme. The glutamine-oxoglutarate amino transferase may be derived from bacterium belonging to the genus Escherichia or Corynebacterium.

Further, the present invention provides a gene encoding glutamine-oxoglutarate amino transferase which comprises a nucleotide sequence corresponding to at least nucleotide numbers of 565 to 6614 of a nucleotide sequence depicted in SEQ ID NO: 7.

Also, the present invention provides a method for producing L-glutamic acid by fermentation, comprising the steps of cultivating, in a liquid medium, a bacterial strain belonging to the genus Corynebacterium which has an ability to produce L-glutamic acid, wherein glutamine-oxoglutarate amino transferase activity is enhanced in a cell of said bacterial strain, producing and accumulating L-glutamic acid in the culture medium, and recovering the L-glutamic acid from the medium.

The present invention will be explained in detail below.

### (1) Bacteria belonging to the genus Corynebacterium having an ability to produce L-glutamic acid

Bacteria belonging to the genus Corynebacterium as referred to herein are a group of microorganisms defined in Bergey's Manual of Determinative Bacteriology, 8th Ed., p. 599 (1974). The bacteria are aerobic, Gram-positive, non-acid-fast bacilli not having the ability to sporulate, and include bacteria which had been classified as bacteria belonging to the genus Brevibacterium but have now been unified into the genus Corynebacterium [see Int. J. Syst. Bacteriol., 41, 255 (1981)] and also include bacteria of the genus Brevibacterium and Microbacterium which are closely related to the genus Corynebacterium. Of such bacteria belonging to the genus Corynebacterium, those mentioned below, which are known as L-glutamic acid-producing bacteria, are most preferred for use in the present invention.
Corynebacterium acetoacidophilum
Corynebacterium acetoglutamicum
Corynebacterium callunae
Corynebacterium glutamicum
Corynebacterium lilium (Corynebacterium glutamicum)
Corynebacterium melassecola
Brevibacterium divaricatum (Corynebacterium glutamicum)
Brevibacterium lactofermentum (Corynebacterium glutamicum)
Brevibacterium saccharolyticum
Brevibacterium immariophilium
Brevibacterium roseum
Brevibacterium flavum (Corynebacterium glutamicum)
Brevibacterium thiogenitalis

Specifically, the following strains of these bacteria are exemplified:
- Corynebacterium acetoacidophilum: ATCC 13870
- Corynebacterium acetoglutamicum: ATCC 15806
- Corynebacterium callunae: ATCC 15991
- Corynebacterium glutamicum: ATCC 13032
- Corynebacterium glutamicum: ATCC 13060
- Brevibacterium divaricatum: ATCC 14020
- Brevibacterium lactofermentum: ATCC 13869
- Corynebacterium lilium: ATCC 15990
- Corynebacterium melassecola: ATCC 17965
- Brevibacterium saccharolyticum: ATCC 14066
- Brevibacterium immariophilium: ATCC 14068
- Brevibacterium roseum: ATCC 13825
- Brevibacterium flavum: ATCC 13826
- Brevibacterium thiogenitalis: ATCC 19240

These strains can be obtained from the American Type Culture Collection (ATCC). A registration number has been assigned to each strain of bacteria. Based on the registration number, anyone can obtain the corresponding strain of bacteria from ATCC. The registration numbers of the strains of bacteria deposited in ATCC are described in the ATCC catalog.

To produce L-glutamic acid using above described bacteria belonging to the genus Corynebacterium, any of the following means is effective.
1. The biotin concentration in the medium is made suboptimal. Refer to S. Okumura, T. Tsugawa, T. Tsunoda and A. Kitai, Nippon Ndgeikagaku Kaishi, 36, 197-203 (1962).
2. A surfactant is added to the medium provided that a sufficient amount of biotin is present. Refer to I. Shiio, H. Otsuka and N. Atsuya, J. Biochem., 53, 333-340 (1963); K. Takinami, H. Okada and T. Tsunoda, Agr. Biol. Chem., 27, 853-863 (1963).
3. Penicillin is added to the medium provided that a sufficient amount of biotin is present. Refer to U.S. Patent 3,080,297; Japanese Patent Publication No. 37-1695 (1962); M. Shibui, T. Kurima, S. Okabe and T. Osawa, Amino Acid and Nucleic Acid, 17, 61-65 (1968).

Also, L-glutamic acid can be produced by a method using mutants derived from the above-described bacteria belonging to the genus Corynebacterium. As examples of the mutant, the following may be mentioned:
Brevibacterium lactofermentum AJ 12745 (FERM-BP 2922); see U.S. Patent No. 5,272,067.
Brevibacterium lactofermentum AJ 12746 (FERM-BP 2923); see U.S. Patent No. 5,272,067.
Brevibacterium flavum AJ 12747 (FERM-BP 2924); see U.S. Patent No. 5,272,067.
Corynebacterium glutamicum AJ 12478 (FERM-BP 2925); see U.S. Patent No. 5,272,067.
Corynebacterium glutamicum ATCC 21492.

In the present invention, it is most preferable to use L-glutamic acid-producing bacterial strains as described above.

### (2) Enhancement of GOGAT activity

GOGAT activity in a bacterial cell can be enhanced by constructing recombinant DNA by ligating a gene fragment that codes for GOGAT with a vector that functions in bacteria belonging to the genus Corynebacterium, transforming a host strain of bacterium belonging to the genus Corynebcterium which has an ability to produce L-glutamic acid by introducing the recombinant DNA to the strain. GOGAT is a heterooligomer comprising large subunit and small subunit each of which is coded by gltB gene and gltD gene, respectively. As a result of increase of copy number of the gene that codes for GOGAT (hereinafter referred to as "gltBD gene") in cells of the transformants, GOGAT activity is enhanced.

As to the gltBD gene, the gene of bacteria belonging to the genus Corynebacterium and also the gene derived from other organisms including Esherichia coli can be used. Nucleotide sequence of the gltBD gene derived from bacteria belonging to the genus Corynebacterium has not been known, but nucleotide sequences of gltBD genes of Esherichia coli K-12 (Gene, 60, 1-11 (1987)) and yeast (Saccharomyces cerevisiae, GenBank accession No. X89221) has been already clarified. Therefore, it is possible to synthesize primers based on the nucleotide sequences of these gltBD genes, and to obtain the gltBD gene of microorganisms such as Esherichia coli K-12 and Brevibacterium lactofermentum ATCC 13869 by the PCR method by using chromosomal DNA prepared from these microorganisms as a template. Such primers may be exemplified by the primers shown in SEQ ID Nos: 3 to 6. The nucleotide sequence of gltBD gene of Brevibacterium lactofermentum ATCC 13869 isolated in Example described later is shown in SEQ ID NO: 7. The gltBD gene of Brevibacterium lactofermentum is novel.

As for plasmids which are used for gene cloning, any plasmid that is replicable in cells of microorganism such as Esherichia may be used, and pBR322, pTWV228, pMW119 and pUC19 are concretely exemplified.

The vector functioning in bacterium belonging to the genus Corynebacterium as referred to herein is, for example, a plasmid which is autonomously replicable in bacteria belonging to the genus Corynebacterium. Specific examples of the vector are mentioned below.
pAM 330 see Japanese Patent Application Laid-Open No. 58-67699 (1983)
pHM 1519 see Japanese Patent Application Laid-Open No. 58-77895 (1983)
pAJ 655 see Japanese Patent Application Laid-Open No. 58-192900 (1983)
pAJ 611 see Japanese Patent Application Laid-Open No. 58-192900 (1983)
pAJ 1844 see Japanese Patent Application Laid-Open No. 58-192900 (1983)
pCG 1 see Japanese Patent Application Laid-Open No. 57-134500 (1982)
pCG 2 see Japanese Patent Application Laid-Open No. 58-35197 (1983)
pCG 4 see Japanese Patent Application Laid-Open No. 57-183799 (1982)
pCG 11 see Japanese Patent Application Laid-Open No. 57-183799 (1982)

In order to prepare recombinant DNA by ligating the gltBD gene which encodes GOGAT and a vector which can function in a cell of bacterium belonging to the genus Corynebacterium, the vector is digested by restriction enzyme(s) corresponding to the termini of the gltBD gene. Ligation is generally performed by using a ligase such as T4 DNA ligase.

To introduce the recombinant DNA prepared as described above to bacterium belonging to the genus Corynebacterium, any known transformation methods can be employed. For instance, employable are a method of treating recipient cells with calcium chloride so as to increase the permeability of DNA, which has been reported for Escherichia coli K-12 [see Mandel, M. and Higa, A., J. Mol. Biol., 53, 159 (1970)]; and a method of preparing competent cells from cells which are at the growth phase followed by introducing the DNA thereinto, which has been reported for Bacillus subtilis [see Duncan, C.H., Wilson, G.A. and Young, F.E., Gene, 1, 153 (1977)]. In addition to these, also employable is a method of making DNA-recipient cells into the protoplast or spheroplast which can easily take up recombinant DNAs followed by introducing the recombinant DNA into the cells, which is known to be applicable to Bacillus subtilis, actinomycetes and yeasts [see Chang, S. and Choen, S.N., Molec. Gen. Genet., 168, 111 (1979); Bibb, M.J., Ward, J.M. and Hopwood, O.A., Nature, 274, 398 (1978); Hinnen, A., Hicks, J.B. and Fink, G.R., Proc. Natl. Sci., USA, 75, 1929 (1978)].

The method of transformation used in embodiments of the present invention is the electric pulse method (refer to Japanese Patent Publication Laid-Open No. 2-207791).

Enhancement of GOGAT activity can also be achieved by introducing multiple copies of the gltBD gene into the chromosomal DNA of the above-described host strains. In order to introduce multiple copies of the gltBD gene in the chromosomal DNA of bacterium belonging to the genus Corynebacterium, the homologous recombination is carried out using a sequence whose multiple copies exist in the chromosomal DNA as targets. As sequences whose multiple copies exist in the chromosomal DNA, repetitive DNA, inverted repeats exist at the end of a transposable element can be used. Also, as disclosed in Japanese Patent Publication Laid-Open No. 2-109985, it is possible to incorporate the gltBD gene into transposon, and allow it to be transferred to introduce multiple copies of the gltBD gene into the chromosomal DNA. By either method, the number of copies of the gltBD gene within cells of the transformant strain increases, and as a result, GOGAT activity is enhanced.

Other than the above-described gene amplification, enhancement of GOGAT activity can also be achieved by substituting the expression regulation sequence such as promoter of the gltBD gene with a more potent one. For example, lac promoter, trp promoter, trc promoter, tac promoter, and P_{R} promoter and P_{L} promoter of lambda phage are known as potent promoters. By substituting the promoter inherent in gltBD gene with these promoters, the expression of gltBD gene is enhanced, thereby enhancing GOGAT activity.

### (3) Production of L-glutamic acid using bacterial strains of the present invention

By using a bacterial strain belonging to the genus Corynebacterium which has an ability to produce L-glutamic acid, wherein GOGAT activity is enhanced in a cell of said bacterial strain, L-glutamic acid can be produced by an ordinary method which uses a common nutrient culture medium containing carbon source, nitrogen source, inorganic salts and other minor organic nutrients such as amino acids and vitamins as necessary. Both of synthetic and natural medium can be used. Any carbon or nitrogen source used for the medium can be employed if it can be used in the bacterial strain to be cultivated.

As carbon sources, sugars such as glucose, glycerol, fructose, sucrose, maltose, mannose, galactose, hydrolysate of starch, and molasses are used, and other organic acids such as acetic acid and citric acid are also used alone or in combination with other carbon sources.

As nitrogen sources, ammonia, ammonium salts such as ammonium sulphate, ammonium carbonate, ammonium chloride, ammonium phosphate and ammonium acetate or nitrate may be used.

As minor organic nutrients, amino acids, vitamins, fatty acids, nucleic acids, and further peptone, casamino acids, yeast extract and soy bean protein hydrolysate which contain these nutrients may be used, and in case of using an auxotrophic mutant which needs amino acids or others for its growth, necessary nutrients should be supplemented.

As inorganic salts, phosphates, magnesium salts, calcium salts, iron salts, manganese salts and the like are used.

As to the cultivation method, it is performed under an aerobic condition while fermentation temperature is controlled at between 20 and 45°C, and pH at between 5 and 9. If the pH value lowers during cultivation, calcium carbonate may be added or the culture medium is neutralized with alkaline substance such as ammonia gas.
By thus cultivating for about ten hours to four days, a significant amount of L-glutamic acid is accumulated in the culture medium.

As to the method for recovering L-glutamic acid from the culture medium after the cultivation, any known method for recovery can be used. For example, the product can be collected by the concentration crystallization method after removal of cell from the culture medium, or by ion-exchange chromatography or the like.

### Brief Description of Drawing

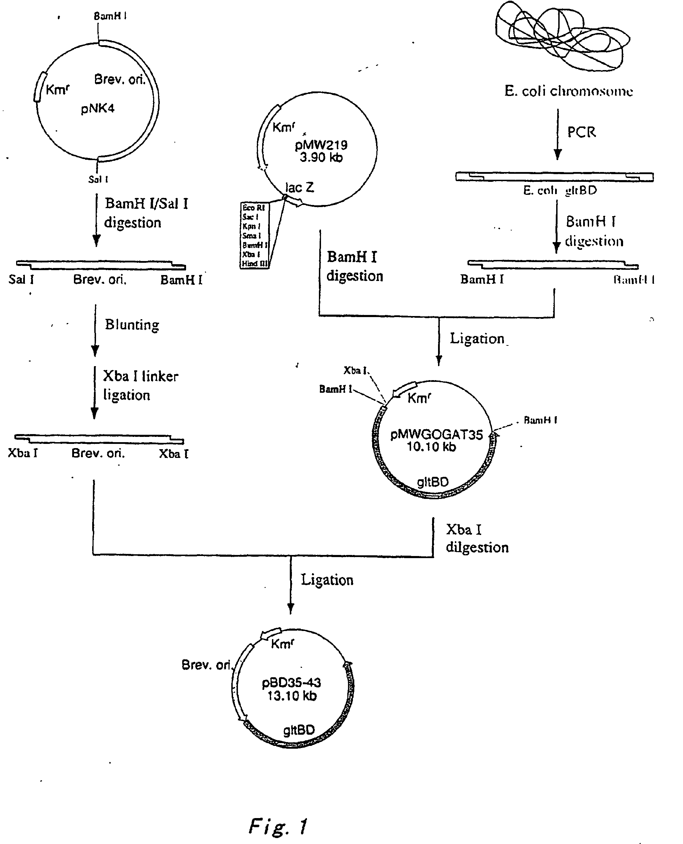

Fig. 1 shows the process for construction of plasmid pBD35-43.

### Description of Preferred Embodiments

The present invention will be more specifically explained below with reference to Examples.

### Example 1

### (1) Cloning of gltBD gene of Esherichia coli K-12

Nucleotide sequence of the gltBD gene of Esherichia coli K-12 has already been known (Gene, 60, 1-11 (1987)). Based on the reported nucleotide sequence, primers shown by SEQ ID NOs: 1 and 2 in the Sequence Listing were synthesized and the gltBD gene was amplified according to the PCR method by using the chromosomal DNA of JM109 strain (produced by Takara Shuzo Co., Ltd.) derived from Esherichia coli K-12 as a template.

Among the synthesized primers, SEQ ID NO: 1 corresponds to a sequence ranging from the 57th to 96th nucleotides in the Figure of nucleotide sequence of the gltBD gene described in Gene, 60, 6 (1987), but the 77th and 78th nucleotides were changed from A to G in the primer, and the recognition site for the restriction enzyme BamHI has been inserted. SEQ ID NO: 2 corresponds to a sequence ranging from the 6261st to 6290th nucleotides in the Figure of nucleotide sequence of the gltBD gene described in Gene, 60, 7 (1987), but the 6380th nucleotide T was changed to G, the 6282nd nucleotide A was changed to T, and the 6284th nucleotide A was changed to C in the primer, thus the recognition site for the restriction enzyme BamHI has been inserted. Also the nucleotide sequence shown in SEQ ID NO: 2 is a sequence which is the oposite strand of the nucleotide sequence ranging from the 6261st to 6290th nucleotides described in the Figure of nucleotide sequence in Gene, 60, 7 (1987), and is directed from the 5' end.

Preparation of chromosomal DNA of Esherichia coli K-12 was performed according to the ordinary method (Seibutsu-kogaku Jikken-sho, edited by Nihon Seibutsu Kougaku-kai, 97-98, Baifu-kan, (1992)). Also, the PCR reaction was performed by using standard reaction conditions described in PCR Technology (Edited by Henry Ehrich, Stockton Press, 1989, page 8).

After the obtained PCR product was purified using the ordinary method, it was treated with a restriction enzyme BamHI, and ligated with pMW219 (produced by Nippon Gene Co., Ltd.), which had been digested with BamHI, using a ligation kit (produced by Takara Shuzo Co., Ltd.), and then transformation was performed using competent cells of Esherichia coli JM109 (produced by Takara Shuzo Co., Ltd.). Then, the cells were plated on L medium (10 g/l Bacto-trypton, 5 g/l Bacto-yeast extract, 15 g/l NaCl, 15 g/l agar, pH 7.2) containing 10 µg/ml of IPTG (isopropyl-β-D-thiogalactopyranoside), 40 µg/ml of X-Gal (5-bromo-4-chloro-3-indolyl-β-D-galactoside) and 25 µg/ml of kanamycin and cultivated over night, then formed white colonies were picked up and isolated as single colonies to obtain transformants.

Plasmids were prepared from the transformants using the alkaline method (Seibutsu-kogaku Jikken-sho, edited by Nihon Seibutsu Kogaku-kai, 105, Baifu-kan, (1992)), and after that, a restriction map of DNA fragment which had been inserted into vectors was prepared, and based on the comparison with reported restriction map of the gltBD gene, a plasmid in which DNA fragment having the restriction map same as the reported map had been inserted was named as pMWGOGAT35.

Further, in order to confirm that the gltBD gene was expressed, pMWGOGAT35 was introduced in Esherichia coli PA340 strain which lacked gdh and gltB and had L-glutamic acid requirement (E. coli Genetic stock center (Yale University, U.S.A.)) using the electroporation method. As a result, the transformant in which pMWGOGAT35 was introduced lost the L-glutamic acid requirement, thereby expression of the gltBD gene was confirmed.

### (2) Construction of a plasmid having the gltBD gene of Esherichia coli K-12 and a replication origin of bacterium belonging to the genus Corynebacterium

Process of construction of plasmid pBD35-43 which has the gltBD gene of Esherichia coli K-12 and a replication origin of bacterium belonging to the genus Corynebacterium is shown in Fig. 1. Concretely, plasmid pHK4 (Japanese Patent Application Laid-Open No. 5-7491) which carries the replication origin (Japanese Patent Application Laid-Open No. Hei 5-7491) derived from plasmid pHM1519 which is autonomously replicable in bacterium belonging to the genus Corynebacterium (Agric. Biol. Chem., 48, 2901-2903 (1984)) was digested with restriction enzymes BamHI and KpnI to obtain a gene fragment including the replication origin, and the obtained fragment was blunt-ended using DNA blunting kit (produced by Takara Shuzo Co., Ltd., Blunting kit), and was then introduced at the XbaI site in the plasmid pMWGOGAT35 to which cloned gltBD gene of Esherichia coli K-12 strain had been inserted using XbaI linker (produced by Takara Shuzo Co., Ltd.). This plasmid was designated as pBD35-43.

### (3) Introduction of pBD35-43 to wild strains of bacteria belonging to the genus Corynebacterium AJ12036 and AJ13029 and estimaion of the obtained transformants by cultivation

Wild strains of bacteria belonging to the genus Corynebacterium AJ12036 (Agric. Biol. Chem., 51, 93-100 (1987)) and AJ13029 were transformed with plasmid pBD35-43 using the electric pulse method (refer to Japanese Patent Publication Laid-Open No. 2-207791), and thus transformants were obtained. The transformant AJ12036/pBD35-43 which was obtained by introducing the plasmid pBD35-43 into the wild strain AJ12036 was cultivated to produce L-glutamic acid under biotin restriction condition as follows. Cells of AJ12036/pBD35-43 strain cultivated on CM2B medium plate containing 25µg/ml of kanamycin were inoculated into culture medium in which 80 g of glucose, 1 g of KH₂PO₄, 0.4 g of MgSO₄·7H₂O, 30 g of (NH₄)₂SO₄, 0.01 g of FeSO₄·7H₄O, 0.01 g of MnSO₄·7H₂O, 15 ml of soybean hydrolysate, 200 µg of thiamine hydrochloride, 60 µg of biotin, 25 mg of kanamycin, and 50 g of CaCO₃ were added in one liter of pure water (adjusted at pH 8.0 using KOH), and the cells were cultivated with shaking at 31.5 °C until sugar in the medium was consumed. Obtained culture was inoculated in the medium of the same composition except that biotin and kanamycin were not added (hereinafter referred to as "biotin restriction medium") at the amount of 5%, then cultivated with shaking at 31.5 °C until sugar in the medium was consumed. As a control, AJ12036 strain was cultured in the same manner as mentioned above. After the completion of cultivation, amount of accumulated L-glutamic acid in the culture medium was measured using Biotech Analyzer AS-210 produced by Asahi Chemical Industry Co., Ltd. Obtained results are shown in Table 1.

**Table 1**

| Bacterial Strains | Accumulation of L-glutamic acid (g/L) |
|---|---|
| AJ 12036 | 34.3 |
| AJ 12036/pBD35-43 | 36.8 |

Also, using a transformant AJ13029/pBD35-43 which had been obtained by introducing the plasmid pBD35-43 into AJ 13029 strain, cultivation for L-glutamic acid production was performed as follows. Cells of AJ13029/pBD35-43 strain cultured on CM2B medium plate containing 25 µg/ml of kanamycin were inoculated into culture medium in which 30 g of glucose, 1 g of KH₂PO₄, 0.4 g of MgSO₄·7H₂O, 30 g of (NH₄)₂SO₄, 0.01 g of FeSO₄·7H₂O, 0.01 g of MnSO₄·7H₂O, 15 ml of soy bean hydrolysate, 200 µg of thiamine hydrochloride, 60 µg of biotin, 25 mg of kanamycin, and 50 g of CaCO₃ were added in one liter of pure water (adjusted at pH 8.0 using KOH), and the culture medium was subjected to cultivation with shaking at 31.5 °C until sugar in the medium was consumed. Obtained culture was inoculated in the medium of the same composition at the amount of 5%, then cultivated with shaking at 37.0 °C until sugar in the medium was consumed. As a control, AJ12029 strain was cultured in the same manner as mentioned above. After the completion of cultivation, amount of accumulated L-glutamic acid in the culture medium was measured using Biotech Analyzer AS-210 produced by Asahi Chemical Industry Co., Ltd. Obtained results are shown in Table 2. AJ12029 strain is a strain which is described in WO96/06180 and has temperature-sensitivity for biotin activity repressing substance. This strain was deposited in National Institute of Bioscience and Human Technology, Agency of Industrial Science and Technology, Ministry of International Trade and Industry (zip code: 305-8566, 1-3 Higashi 1-Chome, Tsukuba-shi, Ibaraki-ken, Japan) under the Budapest Treaty. The deposition number of the strain is FERM BP-5189.

**Table 2**

| Bacterial Strains | Accumulation of L-glutamic acid (g/L) |
|---|---|
| AJ 13029 | 16.5 |
| AJ 13029/pBD35-43 | 17.7 |

Based on the above results, it was clarified that L-glutamic acid-producing bacterium which belong to the genus Corynebacterium and have enhanced GOGAT activity via introduction of the gltBD gene showed increased yield of L-glutamic acid.

### Example 2

### (1) Cloning of the gltBD gene of Brevibacterium lactofermentum ATCC13869

It is desirable to use the gltBD gene originated from bacterium belonging to the genus Corynebacterium in the case of fermentative production of L-glutamic acid using bacterium belonging to the genus Corynebacterium.

Oligonucleotides shown in SEQ ID NOs: 3 and 4 were synthesized according to the nucleotide sequence assumed from the amino acid sequence of the selected region of gltB gene products which are highly homologous between Escherichia coli and yeast. Chromosome DNA of was prepared from Brevibacterium lactofermentum ATCC 13869 using Bacterial Genomic DNA Purification Kit (produced by Advanced Genetic Technologies Corp.). Using the above oligonucleotides as primers and the chromosomal DNA as a template, PCR was performed under the standard reaction conditions described in "PCR Technology" (edited by Henry Ehrich, Stockton Press, 1989, page 8). As a result of agarose gel electrophoresis of the PCR product, it was revealed that a DNA fragment of approximately 1.4 kbp was amplified. Both termini of obtained DNA was sequenced using oligonucleotides of SEQ ID Nos: 3 and 4. Sequencing was performed using DNA Sequencing Kit (produced by Applied Biosystems Co., Ltd.) according to the Sanger method (J. Mol. Biol., 143, 161 (1980)). Determined nucleotide sequence was translated into amino acids, and compared the sequence with the amino acid sequences deduced from gltB genes of Esherichia coli and yeast. As a result, because of high homology, it was concluded that the DNA fragment amplified by the PCR was a part of the gltB gene of Brevibacterium lactofermentum ATCC 13869. Then the chromosomal DNA of Brevibacterium lactofermentum ATCC13869 prepared by the above-described method was digested with EcoRI, BamHI, HindIII, PstI and SalI (produced by Takara Shuzo Co., Ltd.) by the ordinary method and was analyzed by Southern hybridization using the amplified DNA fragment as a probe and the DIG DNA Labeling and Detection Kit (produced by Boeringer Mannheim). As a result, it was found that an approximately 14 kb fragment cleaved by HindIII hybridizes with the probe DNA. Therefore, HindIII fragment of chromosomal DNA of Brevibacterium lactofermentum ATCC13869 prepared by the ordinary method was subjected to the agarose gel electrophoresis, and DNA fragments with approximately 10 kb or larger were recovered using glass powder. The recovered DNA fragment was ligated to vector pMW219 (produced by Nippon Gene Corporation) that had been cleaved with the restriction enzyme HindIII (produced by Takara Shuzo Co., Ltd.) using the Ligation kit (produced by Takara Shuzo Co., Ltd.). Competent cells of Esherichia coli JM109 (produced by Takara Shuzo Co., Ltd.) were transformed by the ligation mixture. Transformants were plated on L medium (10 g/ml of Bacto-Tripton, 5 g/l of Bacto-Yeast extract, 15 g/l of NaCl , 15 g/l of agar, pH 7.2) containing 10 µg/ml of IPTG (isopropyl-β-D-thioglactopyronoside), 40 µg/ml of X-Gal (5-bromo-4-chloro-3-indryl-β-D-galactoside) and 25 µg/ml of kanamycin, and incubated overnight. Thus formed white colonies were picked up and isolated to single colonies, and approximately 1,000 transformants were obtained. Using obtained transformants, plasmids were prepared by the alkaline method (Seibutsu-Kogaku Jikken-sho, edited by Nihon Seibutsu-kogaku Gakkai, 105, Baifu-kan, (1992)). PCR was performed using synthetic oligonucleotides having nucleotide sequences shown in SEQ ID NOs: 5 and 6 which were synthesized according to the sequenced portion in the DNA sequence which was used as a probe, and using above plasmid as a template and primers, respectively, under the above-described condition. Then, the transformants which gave an amplified fragment of approximately 1.3 kbp, the size of which is same with the DNA fragment that was amplified when PCR was performed using above primers and using chromosome of Brevibacterium lactofermentum ATCC 13869 as the template, were selected.

### (2) Determination of nucleotide sequence of the gltBD gene of Brevibacterium lactofermentum ATCC13869

Plasmid DNA prepared from the transformants obtained in (1) by the alkaline method contained a DNA fragment of approximately 14 kbp derived from chromosome of Brevibacterium lactofermentum ATCC13869. In the same manner with the above-described method, nucleotide sequence of the gltBD gene contained in the DNA fragment of approximately 14 kbp derived from chromosome of Brevibacterium lactofermentum ATCC 13869 was determined.

Within the thus determined nucleotide sequence, that of AatII fragment which contains gltBD gene is shown in SEQ ID NO: 7. From the nucleotide sequence, two open reading frame was presumed to exist, and amino acid sequences of the translation products that were deduced from the nucleotide sequence were also shown in SEQ ID NO: 7. That is, the gene which encode two proteins having the amino acid sequences shown in SEQ ID NP: 7 is the gltBD gene of Brevibacterium lactofermentum ATCC 13869. In the sequence, nucleotide numbers 565 to 5094 corresponds to the gltB gene, and nucleotide numbers 5097 to 6614 corresponds to the gltD gene. Amino acid sequences coded by the gltB gene and the gltD gene are shown in SEQ ID Nos: 8 and 9, respectively in this order. Incidentally, since the methionine residue located on the N-termini of the proteins are derived from ATG which is the initiation codon, the residue usually has no relation with the function of proteins in temselves, and it is well known that it will be removed by function of peptidase after translation. Accordingly, in case of the above-described proteins, there is a possibility that the methionine residue may be removed.

The nucleotide sequences and amino acid sequences were compared homology with known sequences. Employed data bases were EMBL and SWISS-PROT. As a result, it was revealed that the DNA shown in SEQ ID NO: 7 is a novel gene in bacteria belonging to the genus Corynebacterium which has homology with the gltBD genes of Esherichia coli, yeast, or the like, which had already reported.

### (3) Preparation of a plasmid carrying the gltBD gene of Brevibacterium lactofermentum ATCC 13869 and the replication origin of bacterium belonging to the genus Corynebacterium

In order to study the effect of amplification of the gltBD gene of Brevibacterium lactofermentum ATCC 13869, a plasmid which carried the gltBD gene of Brevibacterium lactofermentum ATCC 13869 and a replication origin of bacterium belonging to the genus Corynebacterium was constructed. Specifically, the plasmid DNA containing the approximately 14 kbp DNA fragment derived from chromosome of Brevibacterium lactofermentum ATCC 13869 which had been prepared from the transformants obtained in (1) by the alkalile method was cleaved with the restriction enzyme AatII to obtain DNA fragment containing the gltBD gene of Brevibacterium lactofermentum ATCC 13869, and obtained fragment was blunt-ended with the DNA blunting kit (produced by Takara Shuzo Co., Ltd., Blunting kit), followed by being inserted into the site of restriction enzyme SmaI within plasmid pVC7 which carried the replication origin derived from plasmid pAM330 which can autonomously replicate in bacterium belonging to the genus Corynebacterium (Japanese Patent Application Laid-Open No. Sho 58-67699). This plasmid was designated as pVCGOGAT. The plasmid pVC7 was constructed by ligating pHSG399, a vector for E. coli (Cm^{r}; Takeshita, S. et al., Gene, 61, 63-74 (1987)) with pAM330, a cryptic plasmid of Brevibacterium lactofermentum. pAM330 was prepared from Brevibacterium lactofermentum ATCC 13869 strain. pHSG399 was digested with a restriction enzyme resulting one cleavage site, AvaII (produced by Takara Shuzo), blunt-ended by using T4 DNA polymerase, and ligated with pAM330 having been digested with HindIII (produced by Takara Shuzo) and blunt-ended by using T4 DNA polymerase. pVC7 is autonomously replicable in both E. coli and Brevibacterium lactofermentum and has a multiple cloning site originating from pHSG399 and lacZ'.

Further, in order to confirm that the gltBD gene was expressed, pVCGOGAT was introduced into Esherichia coli PA340 strain which lacks gdh and gltB and has L-glutamic acid requirement (E. coli Genetic stock center (Yale University, U.S.A.)) by the electroporation method. As a result, the transformant strain carrying pVCGOGAT did not show L-glutamic acid requirement any longer, and expression of the gltBD gene was thus confirmed.

### (4) Introduction of pVCGOGAT into wild strain of bacterium belonging to the genus Corynebacterium AJ 12036 and estimation of cultivation

Wild strain of bacterium belonging to the genus Corynebacterium AJ 12036 (Agric. Biol. Chem., 51, 93-100 (1987)) was transformed with plasmid pVCGOGAT by the electric pulse method (Japanese Patent Publication Laid-Open No. Hei 2-207791) to obtain a transformant. The transformant AJ 12036/pVCGOGAT which was obtained by introducing plasmid pVCGOGAT into wild strain AJ 12036 was cultivated for L-glutamic acid production under biotin restriction condition as follows. Cells of AJ 12036/pVCGOGAT strain obtained by cultivating on CM2B medium plate containing 5 mg/ml of chloramphenicol were inoculated in culture medium in which 80 g of glucose, 1 g of KH₂PO₄, 0.4 g of MgSO₄·7H₂O, 30 g of (NH₄)₂SO₄, 0.01 g of FeSO₄·7H₄O, 0.01 g of MnSO₄·7H₂O, 15 ml of soybean hydrolysate, 200 µg of thiamine hydrochloride, 60 µg of biotin, 10 mg of cholramphenicol, and 50 g of CaCO₃ were added in one liter of pure water (pH was adjusted at 8.0 with KOH) and cultivated with shaking at 31.5 °C until sugar in the culture medium was consumed.

Obtained culture was inoculated at amount of 5% to the medium of the same composition except that biotin and chloramphenicol were not added (hereinafter referred to as "biotin restriction medium"), and cultivated with shaking at 31.5 °C until sugar in the culture medium was consumed. As a control, a bacterial strain which was prepared by introducing pVC7 into AJ 12036 strain using the above-described method was cultivated in the same method as described above. After cultivation, amount of accumulated L-glutamic acid in the medium was measured using the Biotech Analyzer AS-210 (produced by Asahi Chemical Industry Co., Ltd.). The results are shown in Table 3.

**Table 3**

| Bacterial Strains | Accumulation of L-glutamic acid (g/L) |
|---|---|
| AJ 12036/pVC7 | 33.6 |
| AJ 12036/pVCGOGAT | 37.0 |

### Industrial Applicability

According to the method of the present invention, productivity of L-glutamic acid in bacterial strains which belong to the genus Corynebacterium, have enhanced GOGAT activity and are capable of producing L-glutamic acid can be increased, and thereby making L-glutamic acid production more inexpensive and effectively. And, gltBD gene derived from bacterium belonging to the genus corynebacteium was obtained and effectively used to the glutamate production in bacterium belonging to the genus corynebacteium.

## Claims

1. A bacterial strain belonging to the genus Coryne-bacterium which has the ability to produce and accumulate L-glutamic acid in the medium, wherein the glutamine-oxoglutarate amino transferase activity is enhanced in a cell of said bacterial strain.

2. The strain according to claim 1, wherein the enhancement of the glutamine-oxoglutarate amino transferase activity is caused through amplifying the copy number of a gene encoding glutamine-oxoglutarate amino transferase.

3. The strain according to claim 1, wherein the enhancement of the glutamine-oxoglutarate amino transferase activity is caused through alteration of the expression regulation sequence of a gene encoding glutamine-oxoglutarate amino transferase.

4. The strain according to claim 1, wherein said glutamine-oxoglutarate amino transferase is derived from a bacterium belonging to the genus Escherichia or Corynebacterium.

5. The strain according to claim 2 or 3, wherein said gene encoding glutamine-oxoglutarate amino transferase comprises a nucleotide sequence corresponding to at least nucleotide numbers of 565 to 6614 of a nucleotide sequence depicted in SEQ ID NO: 7.

6. A gene encoding glutamine-oxoglutarate amino transferase which comprises a nucleotide sequence corresponding to at least nucleotide numbers of 565 to 6614 of a nucleotide sequence depicted in SEQ ID NO: 7.

7. A method for producing L-glutamic acid by fermentation, comprising the steps of:
cultivating, in a liquid medium, a bacterial strain belonging to the genus Corynebacterium which has the ability to produce L-glutamic acid, wherein the glutamine-oxoglutarate amino transferase activity is enhanced in a cell of said bacterial strain,
producing and accumulating L-glutamic acid in the culture medium, and
recovering the L-glutamic acid from the medium.

## Patentansprüche

1. Bakterienstamm der Gattung Corynebacterium mit der Fähigkeit zur Produktion und Anhäufung von L-Glutaminsäure in dem Medium, wobei die Glutamin-Oxoglutarat-Aminotransferaseaktivität in einer Zelle des Bakterienstammes erhöht ist.

2. Stamm nach Anspruch 1, wobei die Erhöhung der Glutamin-Oxoglutarat-Aminotransferaseaktivität durch Erhöhung der Kopienzahl eines Gens bewirkt wird, das für Glutamin-Oxoglutarat-Aminotransferase kodiert.

3. Stamm nach Anspruch 1, wobei die Erhöhung der Glutamin-Oxoglutarat-Aminotransferaseaktivität durch Veränderung der Expressionsregulationssequenz eines Gens bewirkt wird, das für Glutamin-Oxoglutarat-Aminotransferase kodiert.

4. Stamm nach Anspruch 1, wobei die Glutamin-Oxoglutarat-Aminotransferase von einem Bakterium der Gattung Escherichia oder Corynebacterium abgeleitet ist.

5. Stamm nach Anspruch 2 oder 3, wobei das Gen, das für Glutamin-Oxoglutarat-Aminotransferase kodiert, eine Nukleotidsequenz enthält, die mindestens den Nukleotidnummern 565 bis 6614 der Nukleotidsequenz der SEQ ID NO: 7 enspricht.

6. Gen, das für Glutamin-Oxoglutarat-Aminotransferase kodiert und eine Nukleotidsequenz enthält, die mindestens den Nukleotidnummern 565 bis 6614 der Nukleotidsequenz der SEQ ID NO: 7 enspricht.

7. Verfahren zur Produktion von L-Glutaminsäure durch Fermentation, welches die folgenden Stufen umfasst:
Kultivieren eines Bakterienstammes der Gattung Corynebacterium, der die Fähigkeit zur Produktion von L-Glutaminsäure hat, in einem Medium, wobei die Glutamin-Oxoglutarat-Aminotransferaseaktivität in einer Zelle des Bakterienstammes erhöht ist,
Produktion und Anhäufung von L-Glutaminsäure in dem Kulturmedium und
Gewinnen der L-Glutaminsäure aus dem Medium.

## Revendications

1. Souche bactérienne appartenant au genre Corynebacterium qui a la capacité de produire et d'accumuler de l'acide L-glutamique dans le milieu, où l'activité glutamine-oxoglutarate amino transférase est augmentée dans une cellule de ladite souche bactérienne.

2. Souche selon la revendication 1 où l'augmentation de l'activité glutamine-oxoglutarate amino transférase est provoquée par amplification du nombre de copies d'un gène codant la glutamine-oxoglutarate amino transférase.

3. Souche selon la revendication 1 où l'augmentation de l'activité glutamine-oxoglutarate amino transférase est provoquée par modification de la séquence de régulation de l'expression d'un gène codant la glutamine-oxoglutarate amino transférase.

4. Souche selon la revendication 1 où ladite glutamine-oxoglutarate amino transférase est dérivée d'une bactérie appartenant au genre Escherichia ou Corynebacterium.

5. Souche selon la revendication 2 ou 3 où ledit gène codant la glutamine-oxoglutarate amino transférase comprend une séquence nucléotidique correspondant à au moins les numéros de nucléotides 565 à 6614 d'une séquence nucléotidique représentée dans SEQ ID NO : 7.

6. Gène codant une glutamine-oxoglutarate amino transférase qui comprend une séquence nucléotidique correspondant à au moins les numéros de nucléotides 565 à 6614 d'une séquence nucléotidique représentée dans SEQ ID NO : 7.

7. Procédé pour produire de l'acide L-glutamique par fermentation, comprenant les étapes de :
culture, dans un milieu liquide, d'une souche bactérienne appartenant au genre Corynebacterium qui a la capacité de produire de l'acide L-glutamique, où l'activité glutamine-oxoglutarate amino transférase est augmentée dans une cellule de ladite souche bactérienne,
production et accumulation d'acide L-glutamique dans le milieu de culture, et
récupération de l'acide L-glutamique à partir du milieu.


### SEQUENCE LISTING

<110> AJINOMOTO CO., INC.
<120> Method for Producing L-Glutamic Acid by Fermentation
<130> EPA-53305
<140> PCT/JP98/03535
   <141> 1998-08-07
<150> JP 9-216906
   <151> 1997-08-12
<160> 9
<170> PatentIn Ver. 2.0
<210> 1
   <211> 30
   <212> DNA
   <213> Artificial Sequence
<220>
   <223> Description of Artificial Sequence:primer for amplifying Escherichia coli gltBD gene
<400> 1
   agccttaaag ataggatcca ttttaatttc 30
<210> 2
   <211> 30
   <212> DNA
   <213> Artificial Sequence
<220>
   <223> Description of Artificial Sequence:primer for amplifying Escherichia coli gltBD gene
<400> 2
   attcatggat ccctcgctta aacttccagc 30
<210> 3
   <211> 17
   <212> DNA
   <213> Artificial Sequence
<220>
   <221> UNSURE
   <222> (3,9,12)
   <223> n=a or c or g or t
<220>
   <223> Description of Artificial Sequence:primer for amplifying Brevibacterium lactofermentum gltBD gene
<400> 3
   ggngarggng gngarga 17
<210> 4
   <211> 18
   <212> DNA
   <213> Artificial Sequence
<220>
   <221> UNSURE
   <222> (1,4,7,)
   <223> n=a or c or g or t
<220>
   <223> Description of Artificial Sequence:primer for amplifying Brevibacterium lactofermentum gltBD gene
<400> 4
   nccnccngtc atrtaytc 18
<210> 5
   <211> 32
   <212> DNA
   <213> Artificial Sequence
<220>
   <223> Description of Artificial Sequence:primer for amplifying Brevibacterium lactofermentum gltBD gene
<400> 5
   aatccacgtg aagctagtgg cagaacaagg cg 32
<210> 6
   <211> 30
   <212> DNA
   <213> Artificial Sequence
<220>
   <223> Description of Artificial Sequence:primer for amplifying Brevibacterium lactofermentum gltBD gene
<400> 6
   acgaatgaac aattcaccac tggttgcgcc 30
<210> 7
   <211> 8734
   <212> DNA
   <213> Brevibacterium lactofermentum
<220>
   <221> CDS
   <222> (565)..(5094)
<220>
   <221> CDS
   <222> (5097)..(6614)
<400> 7
   gacgtcgcgg aggaatgctg ccacgccttc gatttttccg gaatcgtctt tcaggatcgt 60
   gatggagaat tccagggaca ttttagatcc gtctgcacgc actcccggaa cattgagggg 120
   ctcggagccg tagcgggttt caccggattc catgacgtga tcccatccgt cccagtgtgc 180
   cttgcggtgt ttttcgggga tgatgatgtc gagggatttt ccgagtgctt cgccggccgt 240
   gtatccaaag agtttctcgg agccgccgtt ccagagtctg attattccat cgcgggtggc 300
   gtagatgatt gcttcttctg tttcggtgac aagtcgggct gcgatggtgt caaaatcgac 360
   catttttatc ttctttcacg gggtggatag gcgaacatct tctaccatat cctgtgatgt 420
   gtaacacagg agcgtaatct gacctcccgt tttcctatag attgatcgaa agtaaccctt 480
   ttgttacttg cgttgcaggt agtgcgcctg attttcttat tatcgaacga ttgatagaaa 540
   caggattaaa gtgaggtatc ccgc atg aaa cca caa gga ctc tac aac cct    591
                              Met Lys Pro Gln Gly Leu Tyr Asn Pro
                               1               5
   gcg cat gaa cat gac gcc tgc ggt gtg gcg ttt att gcg gat atc cac   639
   Ala His Glu His Asp Ala Cys Gly Val Ala Phe Ile Ala Asp Ile His
    10                  15                  20                  25
   ggt cga ccc agc cgc agc att gtt gat cgt gca ctt gag gcg ctt cgc   687
   Gly Arg Pro Ser Arg Ser Ile Val Asp Arg Ala Leu Glu Ala Leu Arg
                    30                  35                  40
   aac att gac cac cgt ggc gca gcc ggt gca gag aag aac act ggc gat   735
   Asn Ile Asp His Arg Gly Ala Ala Gly Ala Glu Lys Asn Thr Gly Asp
               45                  50                  55
   ggt gcg ggc atc ctc atg cag att ccg gac ggc ttt tat cgt gaa gta    783
   Gly Ala Gly Ile Leu Met Gln Ile Pro Asp Gly Phe Tyr Arg Glu Val
           60                  65                  70
   tct ggc att gag ctt cct gag gca ggg gag tat gcc act ggt att gcg    831
   Ser Gly Ile Glu Leu Pro Glu Ala Gly Glu Tyr Ala Thr Gly Ile Ala
       75                  80                  85
   ttc ttg cct cgc ggt cgc atg gcg atg atg gat gct cag aag gaa att    879
   Phe Leu Pro Arg Gly Arg Met Ala Met Met Asp Ala Gln Lys Glu Ile
   90                  95                  100                 105
   gag cgc atc gca aag caa gaa ggt gcc gat gtg ctt ggt tgg cgc atg    927
   Glu Arg Ile Ala Lys Gln Glu Gly Ala Asp Val Leu Gly Trp Arg Met
                   110                 115                 120
   gtt cct ttt gat tcc cgt gag ctg ggt tcc atg gct gag gag gcg atg    975
   Val Pro Phe Asp Ser Arg Glu Leu Gly Ser Met Ala Glu Glu Ala Met
               125                 130                 135
   cct agt ttc gcg cag att ttc ctt act gtg cct gga aaa tct ggt gaa    1023
   Pro Ser Phe Ala Gln Ile Phe Leu Thr Val Pro Gly Lys Ser Gly Glu
           140                 145                 150
   gat ctt gac cgt gtg atg ttc ttt atc cgt aag cgt tgt gag cgt gag    1071
   Asp Leu Asp Arg Val Met Phe Phe Ile Arg Lys Arg Cys Glu Arg Glu
       155                 160                 165
   ctg ggc acc acc aat ggt cgc gat acg gtg tat ttc ccg tcg cta tct    1119
   Leu Gly Thr Thr Asn Gly Arg Asp Thr Val Tyr Phe Pro Ser Leu Ser
   170                 175                 180                 185
   tca cgc acc atc att tac aaa ggc atg ttg acc act ctg cag ctt gag    1167
   Ser Arg Thr Ile Ile Tyr Lys Gly Met Leu Thr Thr Leu Gln Leu Glu
                   190                 195                 200
   ggc ttc ttt gag gat ctg ggt gat gct cgc ctg gag tcg gcc att gct    1215
   Gly Phe Phe Glu Asp Leu Gly Asp Ala Arg Leu Glu Ser Ala Ile Ala
               205                 210                 215
   att gtg cac tcg cgt ttc tcc acg aac act ttc cca agc tgg ccg ctg    1263
   Ile Val His Ser Arg Phe Ser Thr Asn Thr Phe Pro Ser Trp Pro Leu
           220                 225                 230
   gcg cac ccg tac cgt ttc gtt gcc cac aac ggt gag atc aac act gtg    1311
   Ala His Pro Tyr Arg Phe Val Ala His Asn Gly Glu Ile Asn Thr Val
       235                 240                 245
   cgt ggc aat gaa aac tgg atg cgc gcc cgc gag gcg ctt atc aaa aac    1359
   Arg Gly Asn Glu Asn Trp Met Arg Ala Arg Glu Ala Leu Ile Lys Asn
   250                 255                 260                 265
   gac aag ctg ggc aat ttg agc agc gtg ctg cct atc tgc acc ccg gag    1407
   Asp Lys Leu Gly Asn Leu Ser Ser Val Leu Pro Ile Cys Thr Pro Glu
                   270                 275                 280
   ggc tcg gat acc gcg cgt ttc gac gag gct ttg gag ctt ttg cac ctg    1455
   Gly Ser Asp Thr Ala Arg Phe Asp Glu Ala Leu Glu Leu Leu His Leu
           285                     290                     295
   ggc gga tac tca ctt ccg cat gct gtt gcg atg atg atc cct cag gcg    1503
   Gly Gly Tyr Ser Leu Pro His Ala Val Ala Met Met Ile Pro Gln Ala
       300                     305                     310
   tgg gaa cac aac aag acg ctg agc cct gag ctg cgt gat ttc tac gaa    1551
   Trp Glu His Asn Lys Thr Leu Ser Pro Glu Leu Arg Asp Phe Tyr Glu
   315                     320                     325
   tac cac tct tgt ctg atg gag cca tgg gat ggt cct gca gcg ctg gca    1599
   Tyr His Ser Cys Leu Met Glu Pro Trp Asp Gly Pro Ala Ala Leu Ala
   330                 335                     340                 345
   ttt act gac ggt cgt ttt gtg ggt gcc gtg ctg gac cgt aat ggc ctg    1647
   Phe Thr Asp Gly Arg Phe Val Gly Ala Val Leu Asp Arg Asn Gly Leu
                   350                     355                     360
   cga cct ggg cga atc acc att act gat tcg ggt ttg gtt gtg atg gct    1695
   Arg Pro Gly Arg Ile Thr Ile Thr Asp Ser Gly Leu Val Val Met Ala
               365                     370                     375
   tct gaa tcg gga gtg ttg gac ttg agg gag gag agc gtc gta aag cgt    1743
   Ser Glu Ser Gly Val Leu Asp Leu Arg Glu Glu Ser Val Val Lys Arg
           380                     385                     390
   act cgc gta cag cct gga cgc atg ttc ctt gtt gac acg gcc gag ggt    1791
   Thr Arg Val Gln Pro Gly Arg Met Phe Leu Val Asp Thr Ala Glu Gly
       395                     400                     405
   cgc atc gtt gaa gac gag gaa atc aag cag aaa tta agc gaa gcg cag    1839
   Arg Ile Val Glu Asp Glu Glu Ile Lys Gln Lys Leu Ser Glu Ala Gln
   410                 415                     420                 425
   cca tat ggt gag tgg att cgc gat aat ttt gtg cat ctg gat cgt ctg    1887
   Pro Tyr Gly Glu Trp Ile Arg Asp Asn Phe Val His Leu Asp Arg Leu
                   430                     435                     440
   cct cag aca cgc tac aac tac atg gcg cac tct cgt gct gtg ttg cgt    1935
   Pro Gln Thr Arg Tyr Asn Tyr Met Ala His Ser Arg Ala Val Leu Arg
               445                     450                     455
   cag cgt gtt ttc gga atc act gaa gaa gat gtg gat ttg ttg ctg ctg    1983
   Gln Arg Val Phe Gly Ile Thr Glu Glu Asp Val Asp Leu Leu Leu Leu
           460                     465                     470
   ccg atg gcc cgc cag ggt gct gag gcg att ggt tcc atg ggt tcg gat    2031
   Pro Met Ala Arg Gln Gly Ala Glu Ala Ile Gly Ser Met Gly Ser Asp
       475                     480                     485
   acg cca att gcg gcg cta tcc cag cga cca cgc atg ctt tat gat ttc    2079
   Thr Pro Ile Ala Ala Leu Ser Gln Arg Pro Arg Met Leu Tyr Asp Phe
   490                 495                     500                 505
   ttc gcg cag cgc ttt gct cag gtg aca aac cca ccg ttg gac tct atc    2127
   Phe Ala Gln Arg Phe Ala Gln Val Thr Asn Pro Pro Leu Asp Ser Ile
                   510                     515                     520
   cgc gaa aag cct gtg acc agc atg ttc act ttg ttg ggt gcg cag tct    2175
   Arg Glu Lys Pro Val Thr Ser Met Phe Thr Leu Leu Gly Ala Gln Ser
             525                 530                 535
   gac gtg ctc aat ccg ggt cct gat gcg gcg cga cgt atc cgt ttg gaa     2223
   Asp Val Leu Asn Pro Gly Pro Asp Ala Ala Arg Arg Ile Arg Leu Glu
           540                 545                 550
   tcg ccg atc att gat aac cat gag ctg gcc acc ttg atc aat gcc aac     2271
   Ser Pro Ile Ile Asp Asn His Glu Leu Ala Thr Leu Ile Asn Ala Asn
       555                 560                 565
   gcg cat ggt gag tgg gat tcc ttt ggt gct gct gta att tct ggt ttg     2319
   Ala His Gly Glu Trp Asp Ser Phe Gly Ala Ala Val Ile Ser Gly Leu
   570                 575                 580                 585
   tac cca gtg gct cac cat ggt gcc ggc atg aag gct gcg att gct cgt     2367
   Tyr Pro Val Ala His His Gly Ala Gly Met Lys Ala Ala Ile Ala Arg
                   590                 595                 600
   gtg cgc cgc gag gtt tct gaa gca atc cgc aat ggc aag acg ttg atc     2415
   Val Arg Arg Glu Val Ser Glu Ala Ile Arg Asn Gly Lys Thr Leu Ile
               605                 610                 615
   gtg ctg tcg gat cgt gaa tct gat gag cgc atg gca cct atc cct gcg     2463
   Val Leu Ser Asp Arg Glu Ser Asp Glu Arg Met Ala Pro Ile Pro Ala
           620                 625                 630
   ctg ctg ctg act tcc gct gtg cat cag tac ttg gtg cag caa cgt acc     2511
   Leu Leu Leu Thr Ser Ala Val His Gln Tyr Leu Val Gln Gln Arg Thr
       635                 640                 645
   cgt acc cag tgc tcc ctg gtg gtg gaa tcc ggc gat gcc cgc gag gtt     2559
   Arg Thr Gln Cys Ser Leu Val Val Glu Ser Gly Asp Ala Arg Glu Val
   650                 655                 660                 665
   cat cac ctg gcg atg ctc att ggt ttt ggt gcc gat gcg atc aac ccg     2607
   His His Leu Ala Met Leu Ile Gly Phe Gly Ala Asp Ala Ile Asn Pro
                   670                 675                 680
   tac atg gca ttt gaa acc atc gat gag ctg cgc atg aag ggt cag ttg     2655
   Tyr Met Ala Phe Glu Thr Ile Asp Glu Leu Arg Met Lys Gly Gln Leu
               685                 690                 695
   ggt gat ctt tct ttg gat gag gca tcc cga aac tac atc aag gca gcc     2703
   Gly Asp Leu Ser Leu Asp Glu Ala Ser Arg Asn Tyr Ile Lys Ala Ala
           700                 705                 710
   acc act ggt gtg ctg aag gtg atg tcc aag atg ggc att gca acg gtg     2751
   Thr Thr Gly Val Leu Lys Val Met Ser Lys Met Gly Ile Ala Thr Val
       715                 720                 725
   tct tcg tac cgt ggc gcg cag ctt gct gat gtc acc ggt ctg cat cag     2799
   Ser Ser Tyr Arg Gly Ala Gln Leu Ala Asp Val Thr Gly Leu His Gln
   730                 735                 740                 745
   gat ctc ctg gac aac tac ttc ggt ggc att gct tca cca att tct ggt     2847
   Asp Leu Leu Asp Asn Tyr Phe Gly Gly Ile Ala Ser Pro Ile Ser Gly
                   750                 755                 760
   atc ggt ctg gat gag gtt gca gct gat gtg gaa gct cgt cac cgc agc     2895
   Ile Gly Leu Asp Glu Val Ala Ala Asp Val Glu Ala Arg His Arg Ser
           765                     770                     775
   gca ttt ttg cca cgc cca gaa gag cac gct cac cgc gaa ttg gat ttg    2943
   Ala Phe Leu Pro Arg Pro Glu Glu His Ala His Arg Glu Leu Asp Leu
               780                 785                 790
   ggt ggt gaa tac aag tgg cgc cgc gaa ggt gaa tat cac ctg ttc aac    2991
   Gly Gly Glu Tyr Lys Trp Arg Arg Glu Gly Glu Tyr His Leu Phe Asn
           795                 800                 805
   cca gaa acc atc ttc aag ctg cag cat gca act cgt tct ggc agc tac    3039
   Pro Glu Thr Ile Phe Lys Leu Gln His Ala Thr Arg Ser Gly Ser Tyr
   810                 815                 820                 825
   gag att ttc aag gat tac acc cgc aag gtt gat gat caa tcc act cgc    3087
   Glu Ile Phe Lys Asp Tyr Thr Arg Lys Val Asp Asp Gln Ser Thr Arg
                   830                 835                 840
   ttg ggt act att cgt gga ctg ttt gag ttc agc acg gat cgc aag cca    3135
   Leu Gly Thr Ile Arg Gly Leu Phe Glu Phe Ser Thr Asp Arg Lys Pro
               845                 850                 855
   att tcg gtg tct gag gtg gag ccg gtc agt gag atc gtg aag cgt ttc    3183
   Ile Ser Val Ser Glu Val Glu Pro Val Ser Glu Ile Val Lys Arg Phe
           860                 865                 870
   tcc act ggt gcg atg tct tat ggc tcg att tct gct gaa gcc cat gag    3231
   Ser Thr Gly Ala Met Ser Tyr Gly Ser Ile Ser Ala Glu Ala His Glu
       875                 880                 885
   gtc ttg gcc atc gcc atg aac cga ctg ggc ggt atg tcc aac tcc ggc    3279
   Val Leu Ala Ile Ala Met Asn Arg Leu Gly Gly Met Ser Asn Ser Gly
   890                 895                 900                 905
   gaa ggt ggc gag gac gcc cgc cga ttc gat gtg gaa ccc aac ggt gac    3327
   Glu Gly Gly Glu Asp Ala Arg Arg Phe Asp Val Glu Pro Asn Gly Asp
                   910                 915                 920
   tgg aag cgc tct gcc att aag cag gtg gcc tcg gga cgt ttc ggc gtg    3375
   Trp Lys Arg Ser Ala Ile Lys Gln Val Ala Ser Gly Arg Phe Gly Val
               925                 930                 935
   acc agc cac tac ttg aac aac tgc acc gat att cag atc aag atg gca    3423
   Thr Ser His Tyr Leu Asn Asn Cys Thr Asp Ile Gln Ile Lys Met Ala
           940                 945                 950
   cag ggc gca aag ccc ggt gaa ggt ggc cag ctg cca cca aac aag gtg    3471
   Gln Gly Ala Lys Pro Gly Glu Gly Gly Gln Leu Pro Pro Asn Lys Val
       955                 960                 965
   tac cca tgg gtt gca gaa gtc cgc atc acc acc cca ggc gtt ggt ctg    3519
   Tyr Pro Trp Val Ala Glu Val Arg Ile Thr Thr Pro Gly Val Gly Leu
   970                 975                 980                 985
   att tcc cct cca cca cac cac gat att tac tcc att gag gat ctg gct    3567
   Ile Ser Pro Pro Pro His His Asp Ile Tyr Ser Ile Glu Asp Leu Ala
                   990                 995                 1000
   cag ctg atc cac gac ctg aag aac gct aac cca cgc gca cga atc cac    3615
   Gln Leu Ile His Asp Leu Lys Asn Ala Asn Pro Arg Ala Arg Ile His
               1005                1010                1015
   gtg aag cta gtg gca gaa caa ggc gtg ggc acc gtt gcc gca ggt gtg    3663
   Val Lys Leu Val Ala Glu Gln Gly Val Gly Thr Val Ala Ala Gly Val
           1020                1025                1030
   tcc aaa gca cac gct gat gtg gtg ctt att tcc ggc cac gac ggc gga    3711
   Ser Lys Ala His Ala Asp Val Val Leu Ile Ser Gly His Asp Gly Gly
       1035                1040                1045
   act ggc gca tct cct ttg acc tcc ctg aag cat gcc ggt ggt cca tgg    3759
   Thr Gly Ala Ser Pro Leu Thr Ser Leu Lys His Ala Gly Gly Pro Trp
   1050                1055                1060                1065
   gag ttg ggc ttg gct gaa acc cag caa acg ttg ctg ctc aac ggc ctg    3807
   Glu Leu Gly Leu Ala Glu Thr Gln Gln Thr Leu Leu Leu Asn Gly Leu
                   1070                1075                1080
   cgt gat cgt att cgc gtg cag tgc gat ggt cag ctg aaa act ggc cga    3855
   Arg Asp Arg Ile Arg Val Gln Cys Asp Gly Gln Leu Lys Thr Gly Arg
               1085                1090                1095
   gac gta gtt atc gca gct ctg ctc ggt gcc gaa gaa ttc ggt ttc gcc    3903
   Asp Val Val Ile Ala Ala Leu Leu Gly Ala Glu Glu Phe Gly Phe Ala
           1100                1105                1110
   acc gca ccg ctg gtg gtt gaa ggc tgc atc atg atg cgc gtc tgc cac    3951
   Thr Ala Pro Leu Val Val Glu Gly Cys Ile Met Met Arg Val Cys His
       1115                1120                1125
   ctg gac acc tgc ccg gtg ggt atc gct acc cag aac ccg gat ttg cgt    3999
   Leu Asp Thr Cys Pro Val Gly Ile Ala Thr Gln Asn Pro Asp Leu Arg
   1130                1135                1140                1145
   tcc aag ttc acc ggc aag gct gaa cac gtg gtc aac ttc ttc acc ttc    4047
   Ser Lys Phe Thr Gly Lys Ala Glu His Val Val Asn Phe Phe Thr Phe
                   1150                1155                1160
   atc gcc cag gaa gtc cgt gag tac ttg gca cag ctt ggt ttc cgc tct    4095
   Ile Ala Gln Glu Val Arg Glu Tyr Leu Ala Gln Leu Gly Phe Arg Ser
               1165                1170                1175
   att gat gaa gcc gta gga caa gcc cag gtg ctg cgt aag cgt tcc gga    4143
   Ile Asp Glu Ala Val Gly Gln Ala Gln Val Leu Arg Lys Arg Ser Gly
           1180                1185                1190
   atc cca gct gat tcc cgc gca gca cac ctg gat ttg agc cca att ttc    4191
   Ile Pro Ala Asp Ser Arg Ala Ala His Leu Asp Leu Ser Pro Ile Phe
       1195                1200                1205
   cat cgc cca gaa act cca cac ttc cca act cag gat gtg cgt tgc acc    4239
   His Arg Pro Glu Thr Pro His Phe Pro Thr Gln Asp Val Arg Cys Thr
   1210                1215                1220                1225
   aag acc cag gaa cac agc cta gaa aaa gcc ctg gac aac gca ttt att    4287
   Lys Thr Gln Glu His Ser Leu Glu Lys Ala Leu Asp Asn Ala Phe Ile
                   1230                1235                1240
   gat aag gct tcg gac aca atc acc cgt gcc gca gcg ggt gtg gaa acc    4335
   Asp Lys Ala Ser Asp Thr Ile Thr Arg Ala Ala Ala Gly Val Glu Thr
           1245                1250                1255
   agc att gtt att gat agc tcc atc agc aac gtc aac cgt tca gtt ggc    4383
   Ser Ile Val Ile Asp Ser Ser Ile Ser Asn Val Asn Arg Ser Val Gly
       1260                1265                1270
   acg atg ctg ggt tct gca gtc agc cgc gtg gct ggt gcc caa ggt ttg    4431
   Thr Met Leu Gly Ser Ala Val Ser Arg Val Ala Gly Ala Gln Gly Leu
   1275                1280                1285
   cca gac ggc acc atc acc ttg aat ctt caa ggc tgc gcc ggt aac tcc    4479
   Pro Asp Gly Thr Ile Thr Leu Asn Leu Gln Gly Cys Ala Gly Asn Ser
   1290                1295                1300                1305
   ttt ggc gcg ttc atc cca cga ggc atc acc atc aac ctc acc ggc gat    4527
   Phe Gly Ala Phe Ile Pro Arg Gly Ile Thr Ile Asn Leu Thr Gly Asp
                   1310                1315                1320
   gcc aat gac ttt gtg ggc aag gga tta tct ggc gga aag att gtg atc    4575
   Ala Asn Asp Phe Val Gly Lys Gly Leu Ser Gly Gly Lys Ile Val Ile
           1325                1330                1335
   aag cct tcc gct cag gct ccg aag cag ctg aag aac aat cca aat atc    4623
   Lys Pro Ser Ala Gln Ala Pro Lys Gln Leu Lys Asn Asn Pro Asn Ile
       1340                1345                1350
   att gcc gga aac gtg ctt gga tac ggc gca acc agt ggt gaa ttg ttc    4671
   Ile Ala Gly Asn Val Leu Gly Tyr Gly Ala Thr Ser Gly Glu Leu Phe
   1355                1360                1365
   att cgt ggc cag gtc ggc gaa cgt ttc tgc gtc cgt aac tct ggc gcc    4719
   Ile Arg Gly Gln Val Gly Glu Arg Phe Cys Val Arg Asn Ser Gly Ala
   1370                1375                1380                1385
   acc gca gtg gtt gaa ggt atc gga aac cac ggt tgt gag tac atg act    4767
   Thr Ala Val Val Glu Gly Ile Gly Asn His Gly Cys Glu Tyr Met Thr
                   1390                1395                1400
   ggc ggc cga gtc ctg gtt ttg ggc ccg gtt ggt gag aac ttt ggt gcc    4815
   Gly Gly Arg Val Leu Val Leu Gly Pro Val Gly Glu Asn Phe Gly Ala
           1405                1410                1415
   ggc atg tct ggt ggc att gca tac ctg gct aat tcc ccg gac cta aac    4863
   Gly Met Ser Gly Gly Ile Ala Tyr Leu Ala Asn Ser Pro Asp Leu Asn
       1420                1425                1430
   cag aag atc aat ggc gaa ttg gtg gat gtt gtt cca ctg agc gct gac    4911
   Gln Lys Ile Asn Gly Glu Leu Val Asp Val Val Pro Leu Ser Ala Asp
   1435                1440                1445
   gat ctg acg tgg gct gat gag ctc att gct cgc cac cgc gaa ctc acc    4959
   Asp Leu Thr Trp Ala Asp Glu Leu Ile Ala Arg His Arg Glu Leu Thr
   1450                1455                1460                1465
   gga tcc gag acc aag ctg cgt gca caa gat ttg gtg aaa atc atg ccg    5007
   Gly Ser Glu Thr Lys Leu Arg Ala Gln Asp Leu Val Lys Ile Met Pro
                   1470                1475                1480
   cgc gat ttc caa aaa gta ctc aac atc atc gaa acg gcc cac gct gag    5055
   Arg Asp Phe Gln Lys Val Leu Asn Ile Ile Glu Thr Ala His Ala Glu
             1485                1490                1495
   ggc caa gac cca gca atc aag atc atg gag gca gtg agc ta atg gcc    5102
   Gly Gln Asp Pro Ala Ile Lys Ile Met Glu Ala Val Ser    Met Ala
         1500                1505                1510       1
   gac cca caa gga ttc atc aaa tac tcc cga cgc gag cct gca cac cgc   5150
   Asp Pro Gln Gly Phe Ile Lys Tyr Ser Arg Arg Glu Pro Ala His Arg
             5                   10                  15
   ccg gtc ccg ctg cgc ctc atg gac tac tcc gag gtc tac gaa aag gca   5198
   Pro Val Pro Leu Arg Leu Met Asp Tyr Ser Glu Val Tyr Glu Lys Ala
         20                  25                  30
   ccg gca ggt cag atc gag gaa cag gct gcc cgc tgc atg gat tgc ggt   5246
   Pro Ala Gly Gln Ile Glu Glu Gln Ala Ala Arg Cys Met Asp Cys Gly
     35                  40                  45                  50
   gtc ccg ttc tgc cac gaa ggc tgc cca ctg ggc aac atc atc cct gag   5294
   Val Pro Phe Cys His Glu Gly Cys Pro Leu Gly Asn Ile Ile Pro Glu
                     55                  60                  65
   tgg aat gat ctg gta cgc caa ggt cgg tgg aag gaa gcc tac gat cgc   5342
   Trp Asn Asp Leu Val Arg Gln Gly Arg Trp Lys Glu Ala Tyr Asp Arg
                 70                  75                  80
   ctg cac gcg acc aac aat ttc ccc gag ttc acc ggc cgt ttg tgc ccc   5390
   Leu His Ala Thr Asn Asn Phe Pro Glu Phe Thr Gly Arg Leu Cys Pro
             85                  90                  95
   gca ccc tgc gaa ggc gcc tgc gtg ctc ggc atc aac gat gat tct gtc   5438
   Ala Pro Cys Glu Gly Ala Cys Val Leu Gly Ile Asn Asp Asp Ser Val
         100                 105                 110
   acc atc aaa aac gtt gag ctg gaa atc gtc gaa aaa gca ttc cgc gaa   5486
   Thr Ile Lys Asn Val Glu Leu Glu Ile Val Glu Lys Ala Phe Arg Glu
   115                 120                 125                 130
   ggc tgg gtt cag cct gtc gtc gca tcc atg tcc acc ggc ctg tcc gta   5534
   Gly Trp Val Gln Pro Val Val Ala Ser Met Ser Thr Gly Leu Ser Val
                   135                 140                 145
   gcc gtc gtc ggc tcc ggt ccc gct ggc ctt gcc gcc gcg cag cag ctc   5582
   Ala Val Val Gly Ser Gly Pro Ala Gly Leu Ala Ala Ala Gln Gln Leu
               150                 155                 160
   acc cgc gca ggt cac agc gtg acc gtc ttc gaa cgc gac gac cgc ctc   5630
   Thr Arg Ala Gly His Ser Val Thr Val Phe Glu Arg Asp Asp Arg Leu
           165                 170                 175
   ggc ggc ctc atg cgc tac ggc gtg cca gaa tac aaa atg gaa aac cgc   5678
   Gly Gly Leu Met Arg Tyr Gly Val Pro Glu Tyr Lys Met Glu Asn Arg
       180                 185                 190
   tgg atc gac cgc cgc atc gag caa atg gaa gca gag ggc aca act ttc   5726
   Trp Ile Asp Arg Arg Ile Glu Gln Met Glu Ala Glu Gly Thr Thr Phe
   195                 200                 205                 210
   cag gta ggc acc tcg cca cgc gcc gct gaa cta gcg ctt ttc gac gcg   5774
   Gln Val Gly Thr Ser Pro Arg Ala Ala Glu Leu Ala Leu Phe Asp Ala
               215                     220                     225
   atc ctc ctc gca acc ggc acc cca gtg gcc cgc gaa ctc tca gtt cca    5822
   Ile Leu Leu Ala Thr Gly Thr Pro Val Ala Arg Glu Leu Ser Val Pro
               230                     235                     240
   ggc cac gat ctc aac ggc atc cat gcg gca atg gat tac ctc acc gcc    5870
   Gly His Asp Leu Asn Gly Ile His Ala Ala Met Asp Tyr Leu Thr Ala
           245                     250                     255
   caa aac cgc atc aat gaa ggc gac ggt gaa gtc tct cca atc aac gcc    5918
   Gln Asn Arg Ile Asn Glu Gly Asp Gly Glu Val Ser Pro Ile Asn Ala
       260                     265                     270
   aaa ggc aag aaa gtt gtc atc atc ggt ggc ggc gac acc ggc acc gac    5966
   Lys Gly Lys Lys Val Val Ile Ile Gly Gly Gly Asp Thr Gly Thr Asp
   275                     280                     285                 290
   tgc ttt ggt aca gcg ctg cgc caa gga gcg gaa tca gtc acc caa ttt    6014
   Cys Phe Gly Thr Ala Leu Arg Gln Gly Ala Glu Ser Val Thr Gln Phe
                   295                     300                     305
   gat atc cgc ccc cgc gct cct ttc cag cgc gcc gat tcc acc cca tgg    6062
   Asp Ile Arg Pro Arg Ala Pro Phe Gln Arg Ala Asp Ser Thr Pro Trp
               310                     315                     320
   ccg atg tac ccc aac ctc ttc cgc acc gca acg gct cac gaa gaa ggc    6110
   Pro Met Tyr Pro Asn Leu Phe Arg Thr Ala Thr Ala His Glu Glu Gly
           325                     330                     335
   gaa tac atc atc act ggc gat gaa tca gcc gat gaa atc gca gcc ctg    6158
   Glu Tyr Ile Ile Thr Gly Asp Glu Ser Ala Asp Glu Ile Ala Ala Leu
       340                     345                     350
   ggc ctc gcc gaa cgc gcc gca ggc tcc acg ctt ggt gaa cgt aaa ttt    6206
   Gly Leu Ala Glu Arg Ala Ala Gly Ser Thr Leu Gly Glu Arg Lys Phe
   355                     360                     365                 370
   gct gtc aac acc gtg gaa ttc cac ggc aac aac ggc cac gtc acc gga    6254
   Ala Val Asn Thr Val Glu Phe His Gly Asn Asn Gly His Val Thr Gly
                   375                     380                     385
   ctc acc ggc aac caa atc cga gtt gtc aac ggc aaa cgt gaa cca atc    6302
   Leu Thr Gly Asn Gln Ile Arg Val Val Asn Gly Lys Arg Glu Pro Ile
               390                     395                     400
   gaa ggc acc gaa ttt ccc ttc gaa gca gac ctc gtt ctc gtc gca ctc    6350
   Glu Gly Thr Glu Phe Pro Phe Glu Ala Asp Leu Val Leu Val Ala Leu
           405                     410                     415
   gga ttc acc ggc gca gaa caa ggc gga ttg gca cac gaa cta ggc gta    6398
   Gly Phe Thr Gly Ala Glu Gln Gly Gly Leu Ala His Glu Leu Gly Val
       420                     425                     430
   ggt ttc gac gac cga gga cgc atc ctc cgc gat tcc gaa tac cgc agc    6446
   Gly Phe Asp Asp Arg Gly Arg Ile Leu Arg Asp Ser Glu Tyr Arg Ser
   435                     440                     445                 450
   ccc acc aac tcc cgc gtt tac atc gca ggc gac aac ggc cgt ggc caa    6494
   Pro Thr Asn Ser Arg Val Tyr Ile Ala Gly Asp Asn Gly Arg Gly Gln
                   455                    460                    465
   tcc ctg atc gtg tgg gca atc gcc gaa ggc cgc gca tgc gcc gca gct    6542
   Ser Leu Ile Val Trp Ala Ile Ala Glu Gly Arg Ala Cys Ala Ala Ala
               470                    475                    480
   atc gac gcc gac ctc atg ggt gaa act gca ctt cca gta gca gtt gca    6590
   Ile Asp Ala Asp Leu Met Gly Glu Thr Ala Leu Pro Val Ala Val Ala
           485                    490                    495
   cca cag gac gtg ccg ctg gct gtc taggaggatt ggcgccaaga accactcgaa   6644
   Pro Gln Asp Val Pro Leu Ala Val
       500                    505
   actgcaattt cgagtggttt tgcgtattca gaatttaagg aaatgcttca aaactatcca 6704
   tatttttgaa gcgtactaag gccgagtttc aattagttgc cgatgctcca cgtatgcaca 6764
   ggttggccac tttcttgatt ctgtaaatac tgttgcaaca ttgctgcaag acctgctttt 6824
   cgagcatcgg aacccggcat gcttccgtgt gcttccagct tgttgaggct acgcaactgc 6884
   caggtggcac cgttttggcg ggacttcgcg cgttcggtga tgatgccgag gtattggtcg 6944
   ataaggtctt tatttacttc aagtcgttcc agtccgattc tggcttgagg gatgaggtgc 7004
   tcttggacca gattcgcaac gttgacttgt ccgagggttg gccaggtcat gcgggcgaac 7064
   agccctgaac gtgcaccgga ttggaagttc ttttccgcat cagggaatag gagacgtgac 7124
   cagacagggc ggttttcctc ggcaagatat ttgaccaaac cgtaatagaa agcggcatcg 7184
   gcagtgatgt cgatgggggt gggacctgca ggtaggagac ggttttccag gcgcaggtgg 7244
   gagcgctctt cgcccggtga gtaaatgggt cggttccagc gccacaccgt tccgttgtgc 7304
   aggttgaggt agtggagtgc gggggatttt cccttcatca ttggtgtgcc agacagtgcg 7364
   cgggattctg cgatgagagg ggagaagtag cgtacgtttt cttcgaaaag atcaaaaaca 7424
   ctggtgatcc atcgttcgcc gaaccatact ctggggcgga ctccttgatt cacaagttca 7484
   ggagtacgcg tatcgatggc ttgttgaaac actggaatgc ggctttcatg ccacacccga 7544
   cggcctagga aaagtgggga gttggcggaa agagctgctt gaacaccagc tattgcttgc 7604
   gacgcattcc acgctgcagc aaacttattg ggcgctaatt gcaggtgcaa ctgaatagac 7664
   gtgcaagttg attcgggggc gagatcagtg aaatcgtgga tgatttgttc acggtcggcg 7724
   atgttgatgt gcactaactc gccacgcgat tccatcaccg cattgctcag cgcacggtag 7784
   cggttttctt gggtcatcca tgccggatct tcaagaaatt ccggagtgat ggtgggcagc 7844
   gtgccgatca tcgccacgtt cacattctcc gatgtggcgg ccgcgcgcac gcgacctaga 7904
   cgcgaggtga tgccttgctc gaggcgtcgt aaagcatcgc ctttaacgga tagtggcggg 7964
   tggttcattt caacattaaa gttaccgatc tccgactggt actcatcgtc taaatgggaa 8024
   agcaccgcgt ggcctgccaa agcaggttgc atatgcttat ccacgaggtt gagttccaac 8084
   tcgaggccga tggaaccctg atcctcaaaa tcggaatgct gcaaatgccg atcaaaaatc 8144
   tcaagatctt ccatgagcag ctggcgataa cgcgtgcgct gctgcggagt gtacctgtca 8204
   gtacttactg attcgcccat aacttagagc aaaccacaaa aaatggtgct gggtaaggat 8264
   accaatcagg aattttccac gcgataaacc tgagccacca attgggtacg cgacctgact 8324
   ttgaaaatat ccaacaattg ggacactgtc ttcttcactg tcgctatcgt gaggcctgtg 8384
   gctttggcaa tttggcggtt ggaattgccc tcgcaaacaa gtttcatcac tcgctgctga 8444
   gtcgccgtta attgttcagg aatctgcgtg gttctgggaa gcatggcacg cagacgagtc 8504
   atggccaacg gggaaagcgt ggtgccaccg gacacagcaa tgcggatgat atcgcgaatg 8564
   tcctgggcac ggtcgctctt gacaatgtat gcagctgcac cattggagag catttcaacc 8624
   agagtttcat ctgtagaaaa agcagtcata gtgacaaaag ctggtggctc ggaaagtttg 8684
   cgtacctcag tgagcagctg cctgccgtcc atgtgaggca tttcgacgtc            8734
<210> 8
   <211> 1510
   <212> PRT
   <213> Brevibacterium lactofermentum
<400> 8
   Met Lys Pro Gln Gly Leu Tyr Asn Pro Ala His Glu His Asp Ala Cys
    1               5                  10                  15
   Gly Val Ala Phe Ile Ala Asp Ile His Gly Arg Pro Ser Arg Ser Ile
               20                  25                  30
   Val Asp Arg Ala Leu Glu Ala Leu Arg Asn Ile Asp His Arg Gly Ala
           35                  40                  45
   Ala Gly Ala Glu Lys Asn Thr Gly Asp Gly Ala Gly Ile Leu Met Gln
       50                  55                  60
   Ile Pro Asp Gly Phe Tyr Arg Glu Val Ser Gly Ile Glu Leu Pro Glu
   65                  70                  75                  80
   Ala Gly Glu Tyr Ala Thr Gly Ile Ala Phe Leu Pro Arg Gly Arg Met
                   85                  90                  95
   Ala Met Met Asp Ala Gln Lys Glu Ile Glu Arg Ile Ala Lys Gln Glu
               100                 105                 110
   Gly Ala Asp Val Leu Gly Trp Arg Met Val Pro Phe Asp Ser Arg Glu
           115                 120                 125
   Leu Gly Ser Met Ala Glu Glu Ala Met Pro Ser Phe Ala Gln Ile Phe
       130                 135                 140
   Leu Thr Val Pro Gly Lys Ser Gly Glu Asp Leu Asp Arg Val Met Phe
   145                 150                 155                 160
   Phe Ile Arg Lys Arg Cys Glu Arg Glu Leu Gly Thr Thr Asn Gly Arg
                   165                 170                 175
   Asp Thr Val Tyr Phe Pro Ser Leu Ser Ser Arg Thr Ile Ile Tyr Lys
               180                 185                 190
   Gly Met Leu Thr Thr Leu Gln Leu Glu Gly Phe Phe Glu Asp Leu Gly
           195                 200                 205
   Asp Ala Arg Leu Glu Ser Ala Ile Ala Ile Val His Ser Arg Phe Ser
       210                 215                 220
   Thr Asn Thr Phe Pro Ser Trp Pro Leu Ala His Pro Tyr Arg Phe Val
   225                 230                 235                 240
   Ala His Asn Gly Glu Ile Asn Thr Val Arg Gly Asn Glu Asn Trp Met
                   245                 250                 255
   Arg Ala Arg Glu Ala Leu Ile Lys Asn Asp Lys Leu Gly Asn Leu Ser
               260                 265                 270
   Ser Val Leu Pro Ile Cys Thr Pro Glu Gly Ser Asp Thr Ala Arg Phe
           275                 280                 285
   Asp Glu Ala Leu Glu Leu Leu His Leu Gly Gly Tyr Ser Leu Pro His
       290                 295                 300
   Ala Val Ala Met Met Ile Pro Gln Ala Trp Glu His Asn Lys Thr Leu
   305 310 315 320
   Ser Pro Glu Leu Arg Asp Phe Tyr Glu Tyr His Ser Cys Leu Met Glu
   325 330 335
   Pro Trp Asp Gly Pro Ala Ala Leu Ala Phe Thr Asp Gly Arg Phe Val
   340 345 350
   Gly Ala Val Leu Asp Arg Asn Gly Leu Arg Pro Gly Arg Ile Thr Ile
   355 360 365
   Thr Asp Ser Gly Leu Val Val Met Ala Ser Glu Ser Gly Val Leu Asp
   370 375 380
   Leu Arg Glu Glu Ser Val Val Lys Arg Thr Arg Val Gln Pro Gly Arg
   385 390 395 400
   Met Phe Leu Val Asp Thr Ala Glu Gly Arg Ile Val Glu Asp Glu Glu
   405 410 415
   Ile Lys Gln Lys Leu Ser Glu Ala Gln Pro Tyr Gly Glu Trp Ile Arg
   420 425 430
   Asp Asn Phe Val His Leu Asp Arg Leu Pro Gln Thr Arg Tyr Asn Tyr
   435 440 445
   Met Ala His Ser Arg Ala Val Leu Arg Gln Arg Val Phe Gly Ile Thr
   450 455 460
   Glu Glu Asp Val Asp Leu Leu Leu Leu Pro Met Ala Arg Gln Gly Ala
   465 470 475 480
   Glu Ala Ile Gly Ser Met Gly Ser Asp Thr Pro Ile Ala Ala Leu Ser
   485 490 495
   Gln Arg Pro Arg Met Leu Tyr Asp Phe Phe Ala Gln Arg Phe Ala Gln
   500 505 510
   Val Thr Asn Pro Pro Leu Asp Ser Ile Arg Glu Lys Pro Val Thr Ser
   515 520 525
   Met Phe Thr Leu Leu Gly Ala Gln Ser Asp Val Leu Asn Pro Gly Pro
   530 535 540
   Asp Ala Ala Arg Arg Ile Arg Leu Glu Ser Pro Ile Ile Asp Asn His
   545 550 555 560
   Glu Leu Ala Thr Leu Ile Asn Ala Asn Ala His Gly Glu Trp Asp Ser
   565 570 575
   Phe Gly Ala Ala Val Ile Ser Gly Leu Tyr Pro Val Ala His His Gly
   580 585 590
   Ala Gly Met Lys Ala Ala Ile Ala Arg Val Arg Arg Glu Val Ser Glu
   595 600 605
   Ala Ile Arg Asn Gly Lys Thr Leu Ile Val Leu Ser Asp Arg Glu Ser
   610 615 620
   Asp Glu Arg Met Ala Pro Ile Pro Ala Leu Leu Leu Thr Ser Ala Val
   625 630 635 640
   His Gln Tyr Leu Val Gln Gln Arg Thr Arg Thr Gln Cys Ser Leu Val
   645 650 655
   Val Glu Ser Gly Asp Ala Arg Glu Val His His Leu Ala Met Leu Ile
   660 665 670
   Gly Phe Gly Ala Asp Ala Ile Asn Pro Tyr Met Ala Phe Glu Thr Ile
           675                 680                 685
   Asp Glu Leu Arg Met Lys Gly Gln Leu Gly Asp Leu Ser Leu Asp Glu
       690                 695                 700
   Ala Ser Arg Asn Tyr Ile Lys Ala Ala Thr Thr Gly Val Leu Lys Val
   705                 710                 715                 720
   Met Ser Lys Met Gly Ile Ala Thr Val Ser Ser Tyr Arg Gly Ala Gln
                   725                 730                 735
   Leu Ala Asp Val Thr Gly Leu His Gln Asp Leu Leu Asp Asn Tyr Phe
               740                 745                 750
   Gly Gly Ile Ala Ser Pro Ile Ser Gly Ile Gly Leu Asp Glu Val Ala
           755                 760                 765
   Ala Asp Val Glu Ala Arg His Arg Ser Ala Phe Leu Pro Arg Pro Glu
       770                 775                 780
   Glu His Ala His Arg Glu Leu Asp Leu Gly Gly Glu Tyr Lys Trp Arg
   785                 790                 795                 800
   Arg Glu Gly Glu Tyr His Leu Phe Asn Pro Glu Thr Ile Phe Lys Leu
                   805                 810                 815
   Gln His Ala Thr Arg Ser Gly Ser Tyr Glu Ile Phe Lys Asp Tyr Thr
               820                 825                 830
   Arg Lys Val Asp Asp Gln Ser Thr Arg Leu Gly Thr Ile Arg Gly Leu
           835                 840                 845
   Phe Glu Phe Ser Thr Asp Arg Lys Pro Ile Ser Val Ser Glu Val Glu
       850                 855                 860
   Pro Val Ser Glu Ile Val Lys Arg Phe Ser Thr Gly Ala Met Ser Tyr
   865                 870                 875                 880
   Gly Ser Ile Ser Ala Glu Ala His Glu Val Leu Ala Ile Ala Met Asn
                   885                 890                 895
   Arg Leu Gly Gly Met Ser Asn Ser Gly Glu Gly Gly Glu Asp Ala Arg
               900                 905                 910
   Arg Phe Asp Val Glu Pro Asn Gly Asp Trp Lys Arg Ser Ala Ile Lys
           915                 920                 925
   Gln Val Ala Ser Gly Arg Phe Gly Val Thr Ser His Tyr Leu Asn Asn
       930                 935                 940
   Cys Thr Asp Ile Gln Ile Lys Met Ala Gln Gly Ala Lys Pro Gly Glu
   945                 950                 955                 960
   Gly Gly Gln Leu Pro Pro Asn Lys Val Tyr Pro Trp Val Ala Glu Val
                   965                 970                 975
   Arg Ile Thr Thr Pro Gly Val Gly Leu Ile Ser Pro Pro Pro His His
               980                 985                 990
   Asp Ile Tyr Ser Ile Glu Asp Leu Ala Gln Leu Ile His Asp Leu Lys
           995                 1000                1005
   Asn Ala Asn Pro Arg Ala Arg Ile His Val Lys Leu Val Ala Glu Gln
      1010                 1015                1020
   Gly Val Gly Thr Val Ala Ala Gly Val Ser Lys Ala His Ala Asp Val
   025 1030 1035 1040
   Val Leu Ile Ser Gly His Asp Gly Gly Thr Gly Ala Ser Pro Leu Thr
   1045 1050 1055
   Ser Leu Lys His Ala Gly Gly Pro Trp Glu Leu Gly Leu Ala Glu Thr
   1060 1065 1070
   Gln Gln Thr Leu Leu Leu Asn Gly Leu Arg Asp Arg Ile Arg Val Gln
   1075 1080 1085
   Cys Asp Gly Gln Leu Lys Thr Gly Arg Asp Val Val Ile Ala Ala Leu
   1090 1095 1100
   Leu Gly Ala Glu Glu Phe Gly Phe Ala Thr Ala Pro Leu Val Val Glu
   105 1110 1115 1120
   Gly Cys Ile Met Met Arg Val Cys His Leu Asp Thr Cys Pro Val Gly
   1125 1130 1135
   Ile Ala Thr Gln Asn Pro Asp Leu Arg Ser Lys Phe Thr Gly Lys Ala
   1140 1145 1150
   Glu His Val Val Asn Phe Phe Thr Phe Ile Ala Gln Glu Val Arg Glu
   1155 1160 1165
   Tyr Leu Ala Gln Leu Gly Phe Arg Ser Ile Asp Glu Ala Val Gly Gln
   1170 1175 1180
   Ala Gln Val Leu Arg Lys Arg Ser Gly Ile Pro Ala Asp Ser Arg Ala
   185 1190 1195 1200
   Ala His Leu Asp Leu Ser Pro Ile Phe His Arg Pro Glu Thr Pro His
   1205 1210 1215
   Phe Pro Thr Gln Asp Val Arg Cys Thr Lys Thr Gln Glu His Ser Leu
   1220 1225 1230
   Glu Lys Ala Leu Asp Asn Ala Phe Ile Asp Lys Ala Ser Asp Thr Ile
   1235 1240 1245
   Thr Arg Ala Ala Ala Gly Val Glu Thr Ser Ile Val Ile Asp Ser Ser
   1250 1255 1260
   Ile Ser Asn Val Asn Arg Ser Val Gly Thr Met Leu Gly Ser Ala Val
   265 1270 1275 1280
   Ser Arg Val Ala Gly Ala Gln Gly Leu Pro Asp Gly Thr Ile Thr Leu
   1285 1290 1295
   Asn Leu Gln Gly Cys Ala Gly Asn Ser Phe Gly Ala Phe Ile Pro Arg
   1300 1305 1310
   Gly Ile Thr Ile Asn Leu Thr Gly Asp Ala Asn Asp Phe Val Gly Lys
   1315 1320 1325
   Gly Leu Ser Gly Gly Lys Ile Val Ile Lys Pro Ser Ala Gln Ala Pro
   1330 1335 1340
   Lys Gln Leu Lys Asn Asn Pro Asn Ile Ile Ala Gly Asn Val Leu Gly
   345 1350 1355 1360
   Tyr Gly Ala Thr Ser Gly Glu Leu Phe Ile Arg Gly Gln Val Gly Glu
   1365 1370 1375
   Arg Phe Cys Val Arg Asn Ser Gly Ala Thr Ala Val Val Glu Gly Ile
   1380 1385 1390
   Gly Asn His Gly Cys Glu Tyr Met Thr Gly Gly Arg Val Leu Val Leu
           1395                1400                1405
   Gly Pro Val Gly Glu Asn Phe Gly Ala Gly Met Ser Gly Gly Ile Ala
       1410                1415                1420
   Tyr Leu Ala Asn Ser Pro Asp Leu Asn Gln Lys Ile Asn Gly Glu Leu
   425                 1430                1435                1440
   Val Asp Val Val Pro Leu Ser Ala Asp Asp Leu Thr Trp Ala Asp Glu
                   1445                1450                1455
   Leu Ile Ala Arg His Arg Glu Leu Thr Gly Ser Glu Thr Lys Leu Arg
               1460                1465                1470
   Ala Gln Asp Leu Val Lys Ile Met Pro Arg Asp Phe Gln Lys Val Leu
           1475                1480                1485
   Asn Ile Ile Glu Thr Ala His Ala Glu Gly Gln Asp Pro Ala Ile Lys
       1490                1495                1500
   Ile Met Glu Ala Val Ser
   505                 1510
<210> 9
   <211> 506
   <212> PRT
   <213> Brevibacterium lactofermentum
<400> 9
   Met Ala Asp Pro Gln Gly Phe Ile Lys Tyr Ser Arg Arg Glu Pro Ala
   1               5                   10                  15
   His Arg Pro Val Pro Leu Arg Leu Met Asp Tyr Ser Glu Val Tyr Glu
               20                  25                  30
   Lys Ala Pro Ala Gly Gln Ile Glu Glu Gln Ala Ala Arg Cys Met Asp
           35                  40                  45
   Cys Gly Val Pro Phe Cys His Glu Gly Cys Pro Leu Gly Asn Ile Ile
       50                  55                  60
   Pro Glu Trp Asn Asp Leu Val Arg Gln Gly Arg Trp Lys Glu Ala Tyr
   65                  70                  75                  80
   Asp Arg Leu His Ala Thr Asn Asn Phe Pro Glu Phe Thr Gly Arg Leu
                   85                  90                  95
   Cys Pro Ala Pro Cys Glu Gly Ala Cys Val Leu Gly Ile Asn Asp Asp
               100                 105                 110
   Ser Val Thr Ile Lys Asn Val Glu Leu Glu Ile Val Glu Lys Ala Phe
           115                 120                 125
   Arg Glu Gly Trp Val Gln Pro Val Val Ala Ser Met Ser Thr Gly Leu
       130                 135                 140
   Ser Val Ala Val Val Gly Ser Gly Pro Ala Gly Leu Ala Ala Ala Gln
   145                 150                 155                 160
   Gln Leu Thr Arg Ala Gly His Ser Val Thr Val Phe Glu Arg Asp Asp
                   165                 170                 175
   Arg Leu Gly Gly Leu Met Arg Tyr Gly Val Pro Glu Tyr Lys Met Glu
               180                 185                 190
   Asn Arg Trp Ile Asp Arg Arg Ile Glu Gln Met Glu Ala Glu Gly Thr
           195                 200                 205
   Thr Phe Gln Val Gly Thr Ser Pro Arg Ala Ala Glu Leu Ala Leu Phe
       210                 215                 220
   Asp Ala Ile Leu Leu Ala Thr Gly Thr Pro Val Ala Arg Glu Leu Ser
   225                 230                 235                 240
   Val Pro Gly His Asp Leu Asn Gly Ile His Ala Ala Met Asp Tyr Leu
                   245                 250                 255
   Thr Ala Gln Asn Arg Ile Asn Glu Gly Asp Gly Glu Val Ser Pro Ile
               260                 265                 270
   Asn Ala Lys Gly Lys Lys Val Val Ile Ile Gly Gly Gly Asp Thr Gly
           275                 280                 285
   Thr Asp Cys Phe Gly Thr Ala Leu Arg Gln Gly Ala Glu Ser Val Thr
       290                 295                 300
   Gln Phe Asp Ile Arg Pro Arg Ala Pro Phe Gln Arg Ala Asp Ser Thr
   305                 310                 315                 320
   Pro Trp Pro Met Tyr Pro Asn Leu Phe Arg Thr Ala Thr Ala His Glu
                   325                 330                 335
   Glu Gly Glu Tyr Ile Ile Thr Gly Asp Glu Ser Ala Asp Glu Ile Ala
               340                 345                 350
   Ala Leu Gly Leu Ala Glu Arg Ala Ala Gly Ser Thr Leu Gly Glu Arg
           355                 360                 365
   Lys Phe Ala Val Asn Thr Val Glu Phe His Gly Asn Asn Gly His Val
       370                 375                 380
   Thr Gly Leu Thr Gly Asn Gln Ile Arg Val Val Asn Gly Lys Arg Glu
   385                 390                 395                 400
   Pro Ile Glu Gly Thr Glu Phe Pro Phe Glu Ala Asp Leu Val Leu Val
                   405                 410                 415
   Ala Leu Gly Phe Thr Gly Ala Glu Gln Gly Gly Leu Ala His Glu Leu
               420                 425                 430
   Gly Val Gly Phe Asp Asp Arg Gly Arg Ile Leu Arg Asp Ser Glu Tyr
           435                 440                 445
   Arg Ser Pro Thr Asn Ser Arg Val Tyr Ile Ala Gly Asp Asn Gly Arg
       450                 455                 460
   Gly Gln Ser Leu Ile Val Trp Ala Ile Ala Glu Gly Arg Ala Cys Ala
   465                 470                 475                 480
   Ala Ala Ile Asp Ala Asp Leu Met Gly Glu Thr Ala Leu Pro Val Ala
                   485                 490                 495
   Val Ala Pro Gln Asp Val Pro Leu Ala Val
               500                 505